(19) **Europäisches Patentamt / European Patent Office / Office européen des brevets**

(11) **EP 2 140 884 B1**

(12) **EUROPEAN PATENT SPECIFICATION**

(45) Date of publication and mention
of the grant of the patent:
**09.05.2018 Bulletin 2018/19**

(21) Application number: **08714993.6**

(22) Date of filing: **19.03.2008**

(51) Int Cl.:
*A61K 39/00* [(2006.01)]  *A61P 3/10* [(2006.01)]
*A61P 37/00* [(2006.01)]  *A61K 31/7088* [(2006.01)]

(86) International application number:
**PCT/CN2008/000540**

(87) International publication number:
**WO 2008/116380 (02.10.2008 Gazette 2008/40)**

(54) **A VACCINE PREVENTING AND/OR TREATING AUTOIMMUNE DISEASES**

IMPFSTOFF ZUR PRÄVENTION UND/ODER BEHANDLUNG VON AUTOIMMUNKRANKHEITEN

VACCIN DE PREVENTION ET/OU DE TRAITEMENT DE MALADIES AUTO-IMMUNES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **26.03.2007 CN 200710064769**

(43) Date of publication of application:
**06.01.2010 Bulletin 2010/01**

(73) Proprietor: **China Agricultural University
Haidian District
Beijing
100094 (CN)**

(72) Inventors:
• **WANG, Bin**
  **Beijing 100094 (CN)**
• **JIN, Huali**
  **Beijing 100094 (CN)**
• **KANG, Youmin**
  **Beijing 100094 (CN)**
• **ZHANG, Wenjuan**
  **Beijing 100094 (CN)**
• **YU, Yang**
  **Beijing 100094 (CN)**
• **LI, Jinyao**
  **Beijing 100094 (CN)**

(74) Representative: **Leonard, Thomas Charles et al
Kilburn & Strode LLP
Lacon London
84 Theobalds Road
London WC1X 8NL (GB)**

(56) References cited:
EP-A1- 1 716 863    WO-A1-00/53019
WO-A2-03/051310    CN-A- 101 020 063

• SCHULTZ J G ET AL: "Antibodies from a DNA peptide vaccination decrease the brain amyliod burden in a mouse model of Alzheimer's disease" JOURNAL OF MOLECULAR MEDICINE, SPRINGER VERLAG, DE LNKD-DOI:10.1007/S00109-004-0570-Z, vol. 82, no. 10, 1 October 2004 (2004-10-01), pages 706-714, XP002315951 ISSN: 0946-2716
• URBANEK-RUIZ I ET AL: "Immunization with DNA encoding an immunodominant peptide of insulin prevents diabetes in NOD mice" CLINICAL IMMUNOLOGY, ACADEMIC PRESS, US LNKD-DOI:10.1006/CLIM.2001.5055, vol. 100, no. 2, 1 August 2001 (2001-08-01), pages 164-171, XP002964169 ISSN: 1521-6616
• KANG ET AL: "Co-inoculation of DNA and protein vaccines induces antigen-specific T cell suppression" BIOCHEMICAL AND BIOPHYSICAL RESEARCH COMMUNICATIONS, ACADEMIC PRESS INC. ORLANDO, FL, US LNKD- DOI:10.1016/J.BBRC.2006.12.124, vol. 353, no. 4, 17 January 2007 (2007-01-17), pages 1034-1039, XP005771098 ISSN: 0006-291X
• WEINER ET AL: "Oral tolerance: immune mechanisms and treatment of autoimmune diseases" IMMUNOLOGY TODAY, ELSEVIER PUBLICATIONS, CAMBRIDGE, GB LNKD-DOI:10.1016/S0167-5699(97)01053-0, vol. 18, no. 7, 1 July 1997 (1997-07-01), pages 335-343, XP022014835 ISSN: 0167-5699

- LI R-K ET AL: "A CELL SURFACE/PLASMA MEMBRANE ANTIGEN OF CANDIDA ALBICANS" JOURNAL OF GENERAL MICROBIOLOGY, SOCIETY FOR MICROBIOLOGY, READING, GB, vol. 137, no. 3, 1 January 1991 (1991-01-01), pages 455-464, XP008025647 ISSN: 0022-1287
- JIN H ET AL: "Induction of active immune suppression by coimmunization with DNA- and protein-based vaccines" VIROLOGY, ACADEMIC PRESS,ORLANDO, US LNKD-DOI:10.1016/J.VIROL.2005.03.029, vol. 337, no. 1, 20 June 2005 (2005-06-20) , pages 183-191, XP004902586 ISSN: 0042-6822
- BAECHER-ALLAN CLARE ET AL: "Suppressor T cells in human diseases." THE JOURNAL OF EXPERIMENTAL MEDICINE 2 AUG 2004 LNKD-PUBMED:15280423, vol. 200, no. 3, 2 August 2004 (2004-08-02), pages 273-276, XP002576612 ISSN: 0022-1007
- FURTADO G C ET AL: "Regulatory T cells in spontaneous autoimmune encephalomyelitis." IMMUNOLOGICAL REVIEWS AUG 2001 LNKD-PUBMED:11722629, vol. 182, August 2001 (2001-08), pages 122-134, XP002576613 ISSN: 0105-2896
- KWISSA M ET AL: "Codelivery of a DNA vaccine and a protein vaccine with aluminum phosphate stimulates a potent and multivalent immune response" JOURNAL OF MOLECULAR MEDICINE, SPRINGER VERLAG, DE, vol. 81, no. 8, 16 July 2003 (2003-07-16), pages 502-510, XP002404711 ISSN: 0946-2716
- STEPHENS L A ET AL: "CD25 is a marker for CD4+ thymocytes that prevent autoimmune diabetes in rats, but peripheral T cells with this function are found in both CD25+ and CD25- subpopulations." JOURNAL OF IMMUNOLOGY (BALTIMORE, MD. : 1950) 15 SEP 2000 LNKD- PUBMED:10975823, vol. 165, no. 6, 15 September 2000 (2000-09-15), pages 3105-3110, XP002576615 ISSN: 0022-1767
- GLINKA ET AL: "Protective Regulatory T Cell Generation in Autoimmune Diabetes by DNA Covaccination with Islet Antigens and a Selective CTLA-4 Ligand" MOLECULAR THERAPY, ACADEMIC PRESS, SAN DIEGO, CA, US LNKD-DOI:10.1016/J.YMTHE.2006.03.021, vol. 14, no. 4, 1 October 2006 (2006-10-01), pages 578-587, XP005644842 ISSN: 1525-0016
- KOYAMA KOJI ET AL: "Follicular dysfunction induced by autoimmunity to zona pellucida." REPRODUCTIVE BIOLOGY NOV 2005 LNKD-PUBMED:16372044, vol. 5, no. 3, November 2005 (2005-11), pages 269-278, XP002576616 ISSN: 1642-431X
- RAZ I ET AL: "Immune modulation for prevention of type 1 diabetes mellitus" TRENDS IN BIOTECHNOLOGY, ELSEVIER PUBLICATIONS, CAMBRIDGE, GB LNKD-DOI:10.1016/J.TIBTECH.2005.01.009, vol. 23, no. 3, 1 March 2005 (2005-03-01), pages 128-134, XP025290715 ISSN: 0167-7799 [retrieved on 2005-03-01]
- STEINMAN LAWRENCE: "Immune therapy for autoimmune diseases." SCIENCE (NEW YORK, N.Y.) 9 JUL 2004 LNKD- PUBMED:15247472, vol. 305, no. 5681, 9 July 2004 (2004-07-09), pages 212-216, XP002576614 ISSN: 1095-9203
- ALISON EVERY ET AL: 'Intranasal vaccination with proinsulin DNA induces regulatory CD4+ T cells that prevent experimental autoimmune diabetes.' THE JOURNAL OF IMMUNOLOGY vol. 176, no. 8, 01 April 2006, pages 4608 - 4615, XP055020156 ISSN: 0022-1767
- AMY E. JUEDES ET AL: 'Regulatory T-cells in type 1 diabetes' DIABETES/METABOLISM RESEARCH AND REVIEWS vol. 20, no. 6, 01 November 2004, pages 446 - 451, XP055020221 DOI: 10.1002/dmrr.508 ISSN: 1520-7552
- H.-S. JUN ET AL: "Prevention of autoimmune diabetes by immunogene therapy using recombinant vaccinia virus expressing glutamic acid decarboxylase", DIABETOLOGIA, vol. 45, no. 5, 1 May 2002 (2002-05-01), pages 668-676, XP055206045, ISSN: 0012-186X, DOI: 10.1007/s00125-002-0806-9
- A. GAUVRIT ET AL: "DNA vaccination encoding glutamic acid decarboxylase can enhance insulitis and diabetes in correlation with a specific Th2/3?CD4 T cell response in non-obese diabetic mice", CLINICAL & EXPERIMENTAL IMMUNOLOGY, vol. 137, no. 2, 1 August 2004 (2004-08-01), pages 253-262, XP055206049, ISSN: 0009-9104, DOI: 10.1111/j.1365-2249.2004.02546.x
- RAMIYA V K ET AL: "Antigen based therapies to prevent diabetes in NOD mice", JOURNAL OF AUTOIMMUNITY, LONDON, GB, vol. 9, 1 January 1996 (1996-01-01), pages 349-356, XP002955224, ISSN: 0896-8411, DOI: 10.1006/JAUT.1996.0047

**Description**

Field of Technology

The present invention relates to vaccines for use in preventing and/or treating type 1 diabetes mellitus and oophoritis.

Background

Autoimmune diseases arise from an organism's overactive immune response of autoantigens causing damage to the organism's own tissues. Autoimmune diseases, such as type I diabetes mellitus, multiple sclerosis, rheumatoid arthritis, oophoritis, myocarditis, chronic thyroiditis, myasthenia gravis, lupus erythematosus, Graves disease, Sjogren Syndrome, and Uveal Retinitis, etc. are common diseases. The prevalence rate of autoimmune diseases in China is over 5%. The treatment of autoimmune diseases is typically with immunosuppressants. Clinically, commonly used immunosuppressant and methods of immunosuppressant include the following: the use of chemical medicines such as tacrolimus (Prograf/FK506), ciclosporin A (CsA), mycophenolate mofetil (CellCept/MMF), azathioprine (Aza), Prednisone (Pred), or methylprednisolone (MP); or by the use of antibodies such as antilymphocyte globulin (ALG) or anti-CD4 monoclonal antibody (OKT4). Annual cost of treatment is in the billions of RMB. However, the aforementioned immunosuppressants all have toxic side effects. The improper use of such immunosuppressants can result in the over inhibition of the organism's immune response, resulting in multiple complications. Also, due to their toxic side effects, immunosuppressants can lead to failure of the suppressed organ. Therefore there have been no specifically effective treatments of autoimmune diseases so far.

Type I diabetes mellitus is an autoimmune disease characterized by the destruction of insulin-producing cells in the pancreas islet due to CD4 T cells, CD8 T cells, and macrophages infiltrating the pancreas. It is estimated that about 5% to 10% of North American diabetes mellitus patients have type I (ADA [American Diabetes Mellitus Association]. 1997. Report of the Expert Committee on the Diagnosis and Classification of Diabetes Mellitus. Diabetes Mellitus Care 20:1183-1197; Atkinson MA, Leiter EH. 1999. The NOD Mouse Model of Type I Diabetes Mellitus: As good as it gets? Nature 5:601-604). The pathogenesis is mainly that type I diabetes mellitus results from T lymphocyte cells which destroy the insulin-producing cells in the pancreas islet. It is a disease characterized by the destruction of insulin-producing cells in the pancreas islet due to CD4 T cells, CD8 T cells, and macrophages infiltrating the pancreas (Atkinson MA, Maclaren NK. 1994. The pathogenesis of insulin-dependent diabetes mellitus. N Engl J Med 331:1428-1436; Benoist C, Mathis D. 1997. Autoimmune diabetes mellitus: Retrovirus as trigger, precipitator or marker? Nature 388:833-834; Bjork S. 2001. The cost of diabetes mellitus and diabetes mellitus care. Diabetes mellitus Res Clin Pract 54 (Suppll): 13-18). Prevalence of type I diabetes mellitus is high among European descent, approximately amounting to 2 million patients. One feature of its geographical distribution is that type I diabetes mellitus among Finnish children is 400 times as high as that among Venezuelan children. It is reported that global morbidity rate of type I diabetes mellitus in 2010 will be 40% higher than it was in 1998. The rapid growth suggests that environmental factors are affecting susceptibility genes, and together they cause prevalence of type I diabetes mellitus to go up.

People discovered diabetic insulitis, that is, lymphocyte infiltration of the islets of Langerhans of the pancreas, and later discovered islet cell antibodies (ICA), T-cells that have automatic response to insulin, carboxypeptidase, and heat shock protein in patients with type I diabetes mellitus. As of 1990, Beakkeskov proved that the 64K antibodies existing in blood serum of patients with type I diabetes mellitus are GAD autoantibodies and T cells that have autonomic response, and believed that GAD are the critical antigens for the autoimmune response of type I diabetes mellitus (Immune Modulation for Prevention of Type I diabetes Mellitus. Itamar Razl, Roy Eldor2 and Yaakov Naparstek. TRENDS in Biotechnology 23:128, 2005. Long Xiurong, Du Wenbin, Su Zhongpu, and Wei Qingzheng, GAD-Ab Inspection in Children with Diabetes Mellitus. Chinese Journal of Pediatrics. Vol. 10, 1998).

EP1716863 discloses a T cell immune response inhibitor including a nucleic acid vaccine and the protein antigen expression of said nucleic acid vaccine. Every et al., J Immunol., 176:4608-4615 (2006) discloses intranasal vaccination with proinsulin DNA can induce regulatory CD4+ T cells that prevent experimental autoimmune diabetes. Juedes & von Herrath, Diabetes Metab Res Rev, 20:446-451 (2004) is a review relating to regulatory T-cells in type 1 diabetes.

At present, clinical treatment is predominantly the supply of insulin, but there have been no good methods yet to prevent or postpone occurrence of the disease. There are many approaches to the study of type I diabetes mellitus, with the main goal to resist the occurrence of autoimmune. In the advanced stage, the induction of cell regeneration is one of the methods used.

Disclosure of the Invention

The present invention provides vaccine for use in preventing and/or treating type 1 diabetes mellitus in a mammal, the vaccine comprising a mixture of (a) a protein selected from the group consisting of an autoantigen causing the autoimmune disease and an immunogenic epitope polypeptide of SEQ ID NO:1; and (b) a recombinant eukaryotic expression vector comprising an insert that encodes the autoantigen or the immunogenic epitope polypeptide of the autoantigen, wherein the autoantigen is insulin, and wherein the vaccine stimulates regulatory T cells to inhibit an antigen-specific T cell response.

The present invention also provides a vaccine for use in preventing and/or treating autoimmune oophoritis in a mammal, the vaccine comprising a mixture of (a) a protein consisting of an autoantigen causing the autoimmune disease; and (b) a recombinant eukaryotic expression vector comprising an insert that encodes the autoantigen, wherein the autoantigen is zona pellucida 3, and wherein the vaccine stimulates regulatory T cells to inhibit an antigen-specific T cell response.

The active component of the vaccine preventing and/or treating autoimmune diseases provided by the present invention is: a mixture consisting of a protein antigen causing an autoimmune disease or the epitope polypeptides thereof, and the recombinant eukaryotic vector with the coding genes of an autoantigen or the epitope polypeptides thereof in multiple cloning sites;

The aforementioned autoantigens are insulin or zona pellucida 3 (ZP3).

The aforementioned vaccine preventing and/or treating autoimmune diseases can be the vaccine preventing and/or treating type I diabetes mellitus.

The active component of the said vaccine preventing and/or treating type I diabetes mellitus is any one of the following mixtures:

1) the mixture of a protein autoantigen of type I diabetes mellitus and the recombinant eukaryotic vector with the coding genes of the protein autoantigen of type I diabetes mellitus inserted into multiple cloning sites;
2) the mixture of a protein autoantigen epitope polypeptide of type I diabetes mellitus and the recombinant eukaryotic expression vector with the coding genes of the protein autoantigen epitope polypeptide of type I diabetes mellitus inserted into multiple cloning sites;
3) the mixture of a protein autoantigen of type I diabetes mellitus and the recombinant eukaryotic expression vector with the coding genes of the protein autoantigen epitope polypeptide of type I diabetes mellitus inserted into multiple cloning sites; or
4) the mixture of a protein autoantigen epitope polypeptide of type I diabetes mellitus and the recombinant eukaryotic expression vector with the coding genes of the protein autoantigen of type I diabetes mellitus inserted into multiple cloning sites.

The aforementioned autoantigen of type I diabetes mellitus used in the invention is insulin. Other type I diabetes mellitus autoantigens of the disclosure are GAD and heat shock protein.

Among the coimmune mixtures, the mixture of a protein autoantigen of type I diabetes mellitus and the recombinant eukaryotic expression vector with the coding genes of the protein autoantigen epitope polypeptide of type I diabetes mellitus inserted into multiple cloning sites can be applied. This mixture can produce regulatory T cells as well as inhibit the occurrence of type I diabetes mellitus.

Likewise, another applicable mixture is the mixture of a protein autoantigen epitope polypeptide of type I diabetes mellitus and the recombinant eukaryotic expression vector with the coding genes of the protein autoantigen of type I diabetes mellitus inserted into multiple cloning sites. This mixture can produce regulatory T cells as well as inhibit the occurrence of type I diabetes mellitus.

Either of both mixtures mentioned above is as effective as the mixture of a protein autoantigen epitope polypeptide of type I diabetes mellitus and the recombinant eukaryotic expression vector with the coding genes of the protein autoantigen epitope polypeptide of type I diabetes mellitus inserted into multiple cloning sites.

For any of the mixtures, the insulin can come from humans, dogs, or cats. Human insulin can be used to treat mice with type I diabetes mellitus. Humans, dogs, cats, and mice are very similar in gene sequence. With respect to nucleic acid sequence, mouse insulin and human insulin are 95% identical, cat insulin and human insulin are 84% identical, and dog insulin and human insulin are 89% identical.

The aforementioned GAD (glutamate decarboxylase) can come from human, dogs, or cats. Human GAD can also be used to treat mice with type I diabetes mellitus. In terms of nucleic acid sequence, human and mouse sequences are 90% identical.

The aforementioned heat shock protein can come from human, dogs, or cats.

The aforementioned type I diabetes mellitus protein autoantigen can specifically be human insulin. The aforementioned amino acid sequence of type I diabetes mellitus protein autoantigen's epitope polypeptide is sequence 1, and the polypeptide is named B9-23. For insertion of coding genes of type I diabetes mellitus protein or the coding genes of type I diabetes mellitus protein autoantigen's epitope polypeptide, the eukaryotic expression vector can be mammalian cells' expression vectors, such as pcDNA3.0 or pVAX1, or provax (Tu Yixian, Jin Huali, Zhang Xinyu, Yang Ruoye, Yang Fu, Zhang Fuchun, and Wang Bin. Comparisons of the Expression Efficiencies and Immune Effects of Two Eukaryotic Vectors Encoding CFSV E2 Gene. Journal of China Agricultural University, 2005, 10 (6): 37-41).

The active component of the aforementioned vaccine preventing and/or treating type I diabetes mellitus can be specifically categorized as human insulin protein and pVAX-insulin, or as B9-23 and pcDB9-23.

In the active component of the vaccine preventing and/or treating type I diabetes mellitus, 1) the mass ratio of the protein autoantigen of type I diabetes mellitus and the recombinant eukaryotic vector with the coding genes of the protein autoantigen of type I diabetes mellitus inserted into multiple cloning sites is 1:5-5:1; and the optimum ratio is 1:1-1:2;

2) the mass ratio of the protein autoantigen epitope polypeptide of type I diabetes mellitus and the recombinant eukaryotic expression vector with the coding genes of the protein autoantigen epitope polypeptide of type I diabetes mellitus inserted into multiple cloning sites is 1:5-5:1; and the optimum ratio is 1:1-1:2;

3) the mass ratio of the protein autoantigen of type I diabetes mellitus and the recombinant eukaryotic expression vector with the coding genes of the protein autoantigen epitope polypeptide of type I diabetes mellitus inserted into multiple cloning sites is 1:5-5:1; and the optimum ratio is 1:1-1:2; and

4) the mass ratio of the protein autoantigen epitope polypeptide of type I diabetes mellitus and the recombinant eukaryotic expression vector with the coding genes of the protein autoantigen of type I diabetes mellitus inserted into multiple cloning sites is 1:5-5:1; and the optimum ratio is 1:1-1:2.

The aforementioned vaccine preventing and/or treating type I diabetes mellitus can be introduced, among other things, by injecting, spraying, dropping into nose, dropping into eyes, penetrating, absorbing, physical or chemical mediating, into the organism, including into muscular, intradermal, subcutaneous, venous, or mucosal tissues; or introduced into the organism by being mixed or wrapped by other substances.

Dosage of the aforementioned vaccine preventing and/or treating type I diabetes mellitus is normally $200\mu$g-10mg active component/kg body weight/time. The vaccine is to be taken once every 7 to 30 days, and it normally requires a 2 to 5 doses.

Experiments with mice prove that both the mixture of the protein autoantigen of type I diabetes mellitus and the recombinant eukaryotic vector with the coding genes of the protein autoantigen of type I diabetes mellitus inserted into multiple cloning sites and the mixture of protein autoantigen epitope polypeptide of type I diabetes mellitus and the recombinant eukaryotic expression vector with the coding genes of the protein autoantigen epitope polypeptide of type I diabetes mellitus inserted into multiple cloning sites are able to inhibit proliferation of mouse T cells, induce the production of immunosuppressants, and effectively prevent the occurrence of type I diabetes mellitus. Likewise, the approach and method of the said vaccine can be used for other autoimmune diseases which are triggered by autoantigens.

Descriptions of Figures:

Fig. 1a is the enzyme digestion results of EcoR I and Xho I of pVAX-insulin.

Fig. 1b is the enzyme digestion results of EcoR I and Xho I of pcDB9-23 vector.

Fig. 1c is the expression of pVAX-insulin in BHK21 cells by RT-PCR analysis.

Fig. 2a is the increase of T cells in pcDB9-23 and B9-23 coimmune groups simulated for 48 hours.

Fig. 2b is the increase of T cells in pcDB9-23 and B9-23 coimmune groups simulated for 96 hours.

Fig. 3 a is the increase of T cells in each immune group simulated for 48 hours.

Fig. 3b is the comparison of increase of T cells in each immune group from Fig. 3a.

Fig. 3c is the comparison of increase of T cells in different immune groups with different dosages of the vaccine.

Fig. 4 is the comparison experiment of preventing mice with NOD (non-obese diabetes) from occurrence of the disease.

Fig. 5 is the HE stain results of the pancreas section of mice with NOD.

Fig. 6a is mice MZP3 gene sequence and the nucleic acid homology analysis of Marmosets, human, Canis familiaris, felis catus, Sus scrofa, and Bos taurus.

Fig. 6b is mice MZP3 gene sequence and amino acid sequence homology analysis of Marmosets, human, Canis familiaris, felis catus, Sus scrofa, and Bos taurus.

Fig. 7 is the identification and expression analysis of eukaryon expression vector pcDmzp3. (a) Enzyme digestion analysis of the vector by EcoR I and Xho. M: DNA standard molecular weight (2000bp, 1000bp, 750bp, 500bp, 250bp, and 100bp); 1, 2: enzyme digestion of recombinant plasmid pcDmzp3 by EcoR I and Xho I. (b) Analysis of pcDmzp3 expression in BHK21 cell with RT-PCR. M: DNA standard molecular weight (2000bp, 1000bp, 750bp, 500bp, 250bp, and 100bp); 1: pcDmzp3 transfected cells MZP3 expression; 2: untransfected cells for contrast.

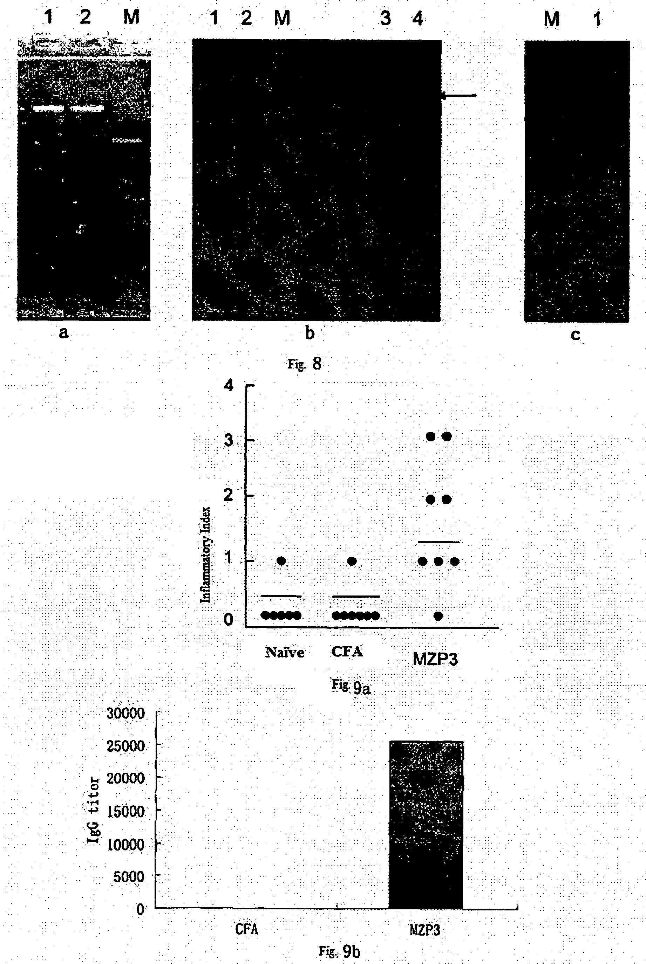

Fig. 8 is the identification, protein expression, and purification of prokaryotic expression vector pGEX-4T-1/MZP3. (a) Enzyme digestion identification of recombinant plasmid pGEX-4T-1/MZP3. M: DNA standard molecular weight (2000bp, 1000bp, 750bp, 500bp, 250bp, and 100bp); 1, 2: enzyme digestion of recombinant plasmid pGEX-4T-1/MZP3 by EcoR I and Xho I. (b) Protein induction expression. M: Protein standard molecular weight; 1: whole cell protein without IPTG induction; 2: IPTG-inducted whole cell protein; 3: ultrasonicated supernatant protein; 4: ultrasonicated precipitated protein, (c) Purified MZP3 protein. M: Protein standard molecular weight; 1: purified protein sample. Arrows point to target bands.

Fig. 9a is the morbidity rate and severity of autoimmune oophoritis. C57BL/6 mice belong to the negative control group with 100μl of PBS injected on the sole and muscle, and the mice belong to the adjuvant group with only 100μl of complete Freund's adjuvant (CFA) injected, and the remaining mice belong to the MZP3 group injected with CFA emulsion containing 100μg of MZP3 protein.

Fig. 9b is the antibody titer (with 3 mice per group). C57BL/6 mice have 100μg of MZP3 protein and CFA injected on the sole and muscle in contrast to mice injected with only CFA. After 14 days from the injection, blood serum is collected, the antibody inspection is done by ELISA, and antibody titer is calculated based on OD values.

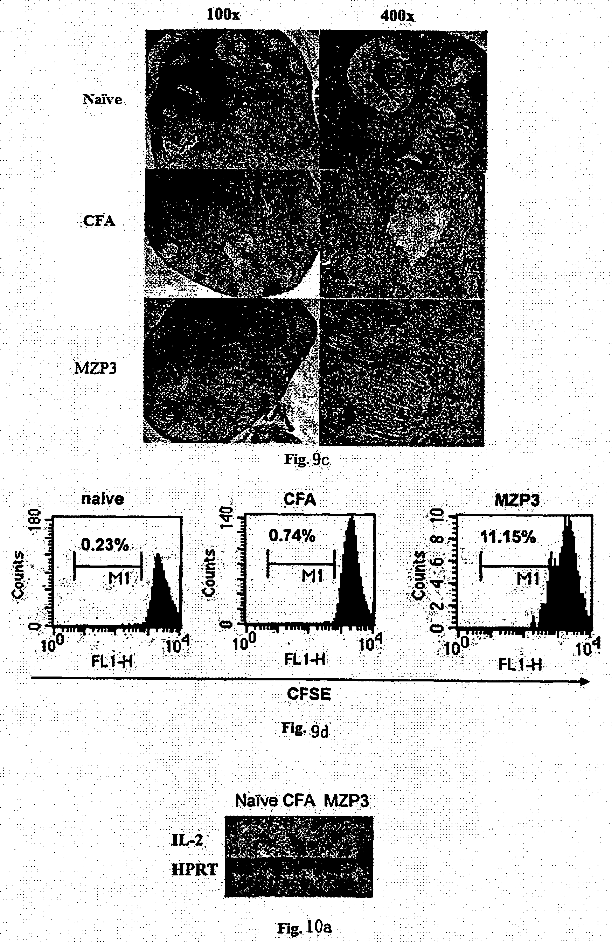

Fig. 9c is the histological change of the ovaries that are examined 14 days after injection. C57BL/6 mice belong to the negative control group with 100μl of PBS injected on the sole and muscle, and the mice belong to the adjuvant group with only 100μl of complete Freund's adjuvant (CFA) injected, and the remaining mice belong to the MZP3 group injected with CFA emulsion containing 100μg of MZP3 protein. The negative control group's ovarian tissues remain normal; for the ovarian tissues of CFA-injected mice, their ovaries do not show inflammatory reaction; the long arrows (with tails) indicate growing ovarian follicles, the arrows without tails indicate the original ovarian follicles (magnification is 100 times and 400 times respectively); for the MZP3 group, inflammatory cells infiltration happens to the ovaries and there is severe loss of eggs.

Fig. 9d is the reaction of lymphocytes from popliteal lymph nodes to MZP3 protein specific T cells. C57BL/6 mice belong to the negative control group with 100μl of PBS injected on the sole and muscle, and the mice belong to the adjuvant group with only 100μl of complete Freund's adjuvant (CFA) injected, and the remaining mice belong to the MZP3 group injected with CFA emulsion containing 100μg of MZP3 protein. T cells are separated from mice (3 mice per group), MZP3 protein is used externally as a specific antigen for stimulation, and inspection is done by means of CFSE. The percentages in the figure are proliferation levels. The bigger the value is, the higher the level of proliferation reaches. M1 represents the percentage of proliferation.

Fig. 10a is the expression of cytokine IL-2. C57BL/6 mice belong to the negative control group (Naive) with 100μl of PBS injected on the sole and muscle, and the mice belong to the adjuvant group with only 100μl of CFA injected, and the remaining mice belong to the MZP3 group injected with CFA emulsion containing 100μg of MZP3 protein.

Fig. 10b is the expression of INF-γ analyzed by flow cytometry. 14 days after the injection, T cells separate from the spleen and popliteal lymph node of mice, and MZP3 protein is used externally to simulate for 8 hours. The expression of CD4+ cell INF-γ and the expression of total cell IL-12 are analyzed by means of intracellular staining. Percentage of positive cells is shown in the figure, a) lymph node; b) spleen.

Fig. 10c is the expression IL-12 analyzed by flow cytometry. After 14 days from the injection, T cells separate from the spleen and popliteal lymph node of mice, and MZP3 protein is used externally to simulate for 8 hours. The expression of CD4+ cell INF-$\gamma$ and the expression of total cell IL-12 are analyzed by means of intracellular staining. Percentage of positive cells is shown in the figure, a) lymph node; b) spleen.

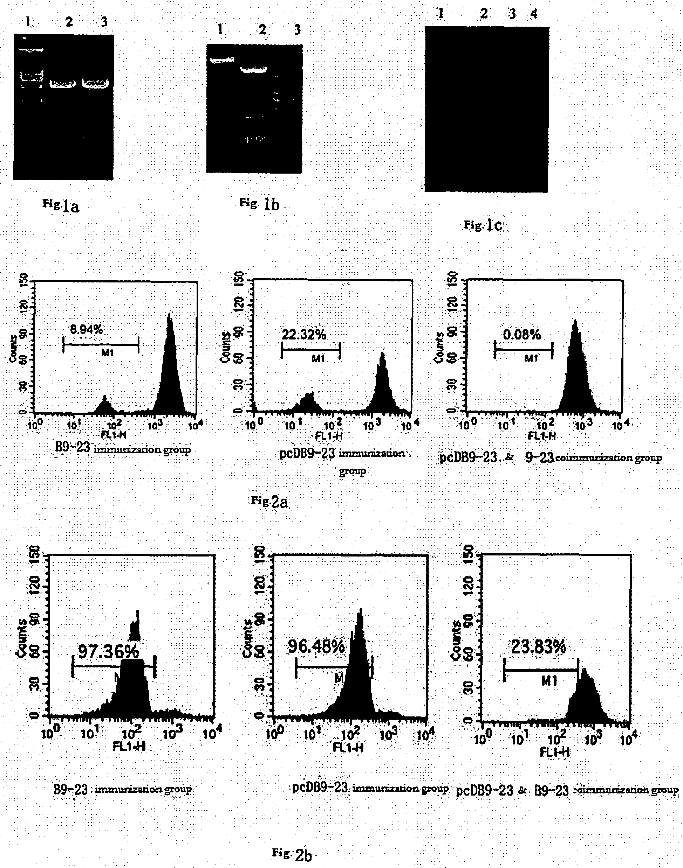

Fig. 1 1a is comparison with the control group, showing the reduction of the coimmune pcDmzp3 and MZP3 protein group's AOD.

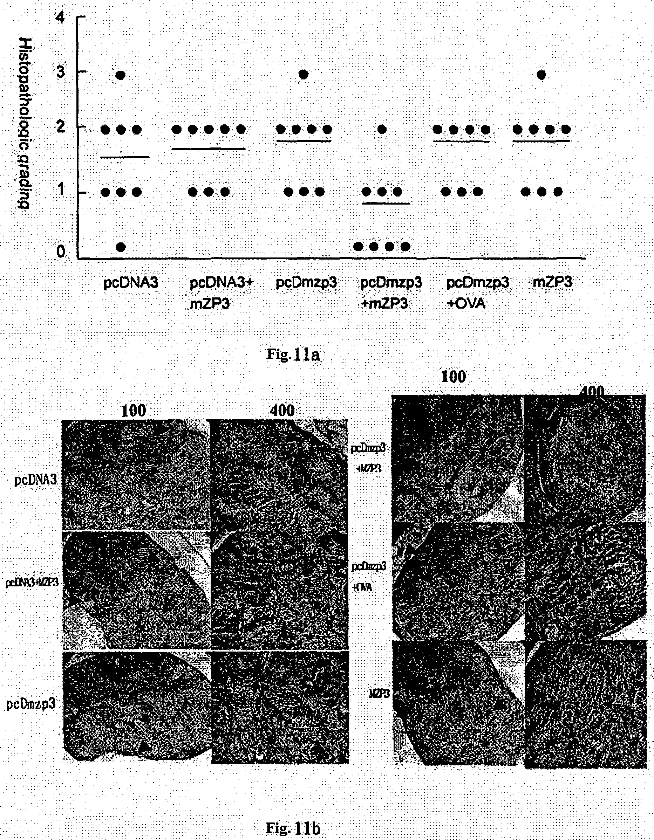

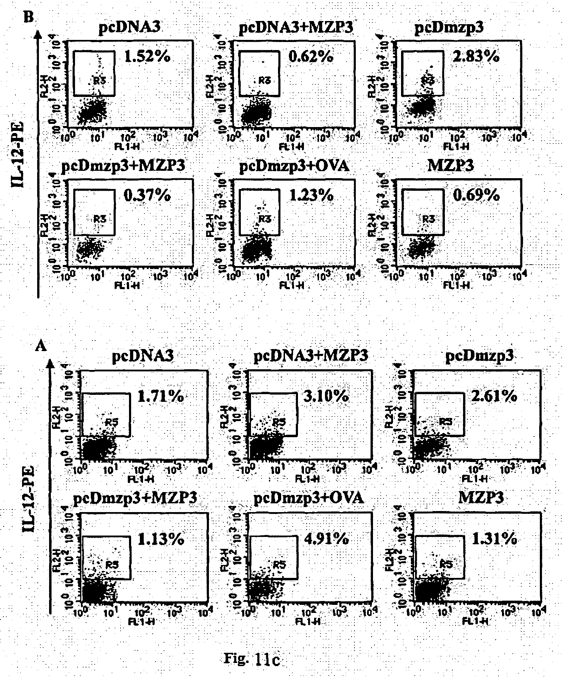

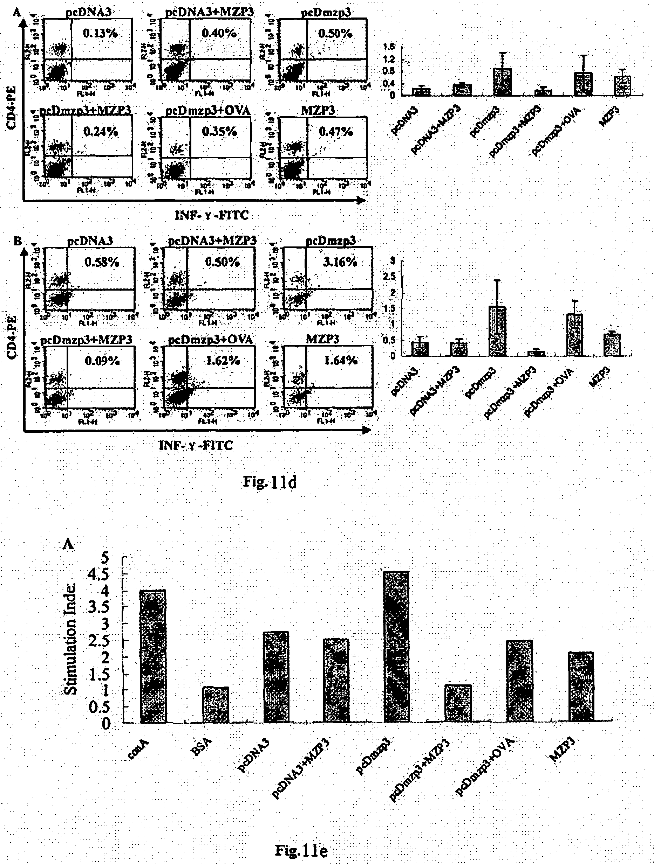

Fig. 11b is histological examination of the ovarian tissues on the seventh day from the second immunization.

Fig. 11c is the expression of cytokine IL-12 examined by flow cytometry: A) lymph node; B) spleen.

Fig. 11d is the expression of cytokine INF-$\gamma$ examined by flow cytometry: A) lymph node; B) spleen.

Fig. 11e is the antigen-specific immunosuppression induced by coimmune DNA and protein. The coimmune antigen matched DNA and mismatched DNA as well as the increase of T cells of mice are compared. On the seventh day from the second immunization, T cells are separated, specific antigen MZP3 protein is used externally for stimulation again. BSA is unrelated protein control, Con A is positive control, MTT approach is used for examination, and index of simulation is used for expression.

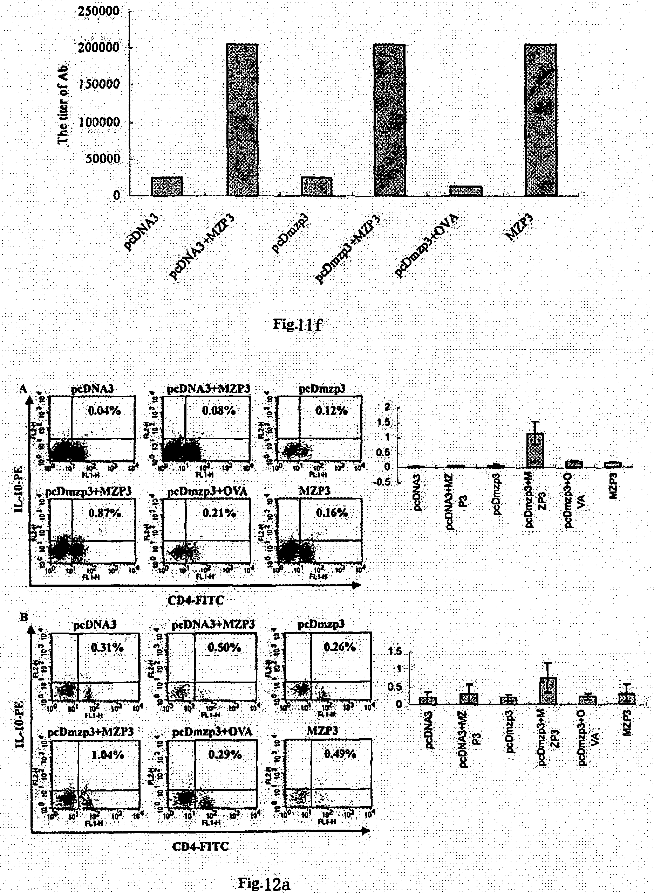

Fig. 11f is examination of antibody level in blood serum by means of ELISA.

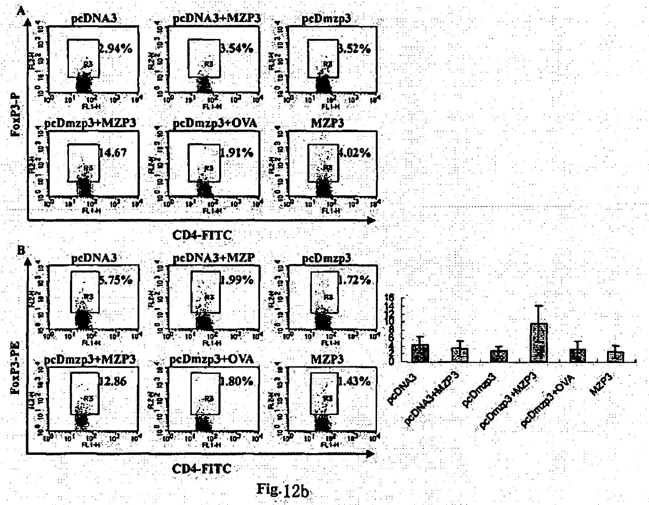

Fig. 12a is the expression of regulatory cells, IL-10, induced by coimmunization. On the seventh day from the second immunization, T cells are separated; the expressions of cytokines IL-10 and FoxP3 are analyzed by flow cytometry. A) lymph node; B) spleen.

Fig. 12b is the expression of regulatory cell, FoxP2, induced by coimmunization. On the seventh day from the second immunization, T cells are separated; the expressions of cytokines IL-10 and FoxP3 are analyzed by flow cytometry. A) lymph node; B) spleen.

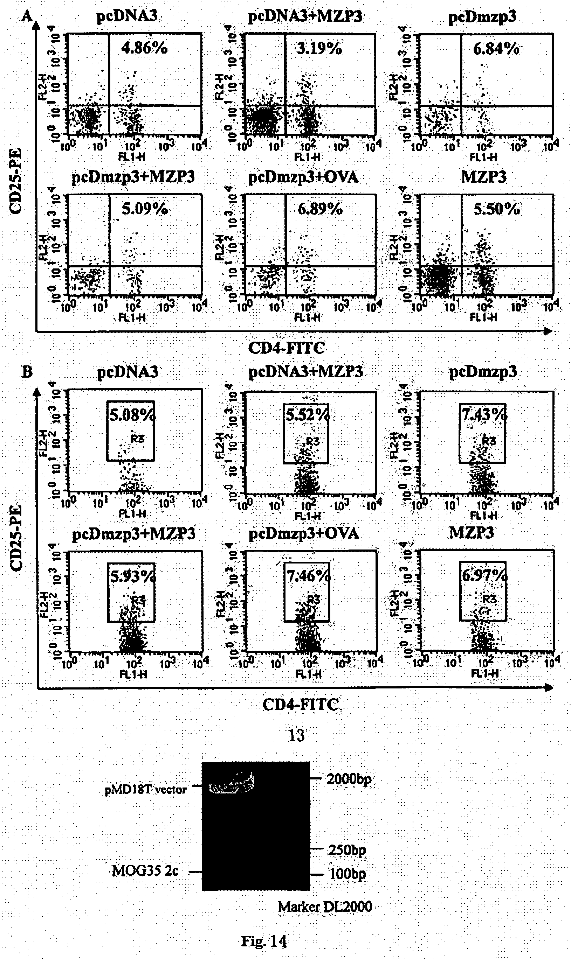

Fig. 13 shows the number of CD4$^+$ and CD25$^+$ cells remain unchanged by coimmunization. T cells are separated by immunizing different groups of mice. Anti-CD4 and anti-CD25 monoclonal antibodies are stained, and analysis is done by flow cytometry. A) lymph node; B) spleen.

Fig. 14 is the enzyme digestion result of plasmid T-MOG352c.

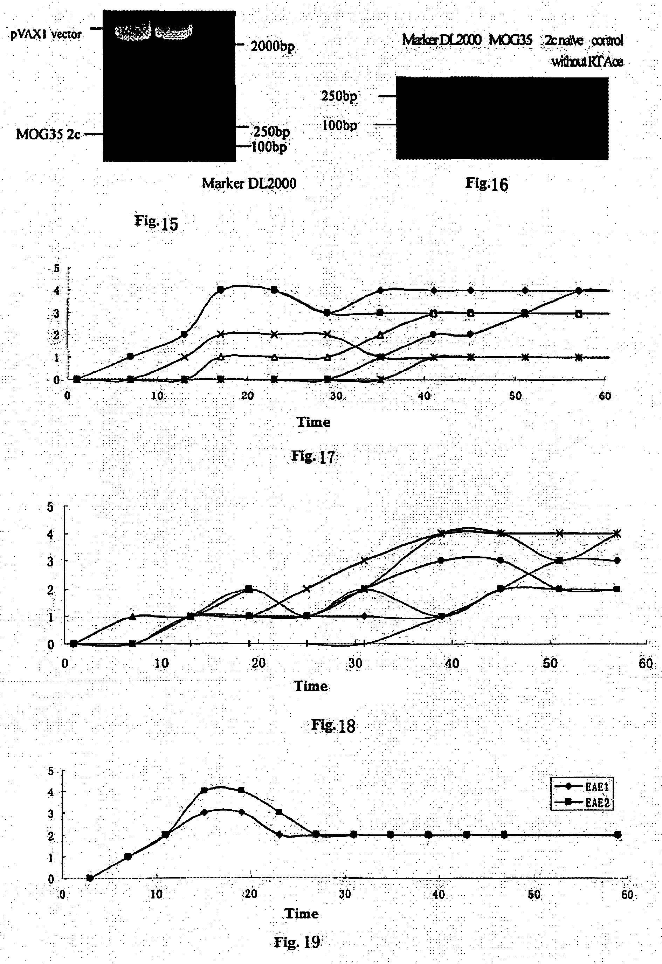

Fig. 15 is the enzyme digestion result of plasmid pVAXMOG352c.

Fig. 16 is the transient expression result of plasmid pVAXMOG352c in BHK21 cell.

Fig. 17 is the morbidity of EAE model where the mice are induced by immunization. The vertical coordinate is the index of morbidity, and the horizontal coordinate is the number of days.

Fig. 18 is the morbidity of negative mice immunized with 200$\mu$g of CFA emulsified MOG antigen after an adoptive transfer of T cells from the spleen of morbid mice. The vertical coordinate is the index of morbidity, and the horizontal coordinate is the number of days.

Fig. 19 is the morbidity of negative mice immunized with 200$\mu$g of CFA emulsified MOG antigen after adoptive transfer of T cells from the lymph node of morbid mice. The vertical coordinate is the index of morbidity, and the horizontal coordinate is the number of days.

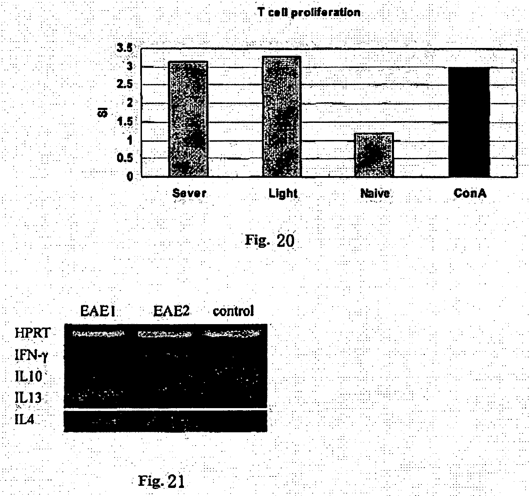

Fig. 20 is the increase of T cells specific to MOG autoantigen after the disease is induced in the mice.

Fig. 21 is about some cytokines inside the T cells specific to MOG autoantigen after the disease is induced in the mice.

The Best Methods to Implement the Invention

Unless otherwise described, the experimental methods in the following embodiments are conventional methods.

Embodiment 1: the vaccines preventing and/or treating type I diabetes mellitus

This embodiment involves four vaccines that prevent and/or treat type I diabetes mellitus: 1) the vaccine consisting of human insulin protein (by Sigma Company, 1-9278) and pVAX-insulin, 2) the vaccine consisting of 9-23 protein (B9-23) on the B chain of insulin and pcDB9-23, 3) the vaccine consisting of human insulin protein (by Sigma Company, 1-9278) and pcDB9-23), and 4) the vaccine consisting of B9-23 and pVAX-insulin.

The amino acid sequence of B9-23 is: S H L V E A L Y L V C G ERG (sequence 1). The 9-23 protein (B9-23) on B chain of insulin is synthesized by Beijing Aoke Company.

Among them, pVAX-insulin and pcDB9-23 are constructed through the following procedures:
Obtain human pancreatic tissues, use the RNA reagent extraction kit purchased from Takara Company, fully grind it in TrizoL reagent, and extract total RNA following the instruction. Design primers according to published gene sequences. The sequences of primers are:

Primers for amplification of human insulin:

mInsulinp1 atggccctgttggtgcacttcctac

mInsulinp2 ttagttgcagtagttctccagctgg

Primers for amplification of polypeptide B9-23 composed of the ninth to twenty-third amino acid residues of the amino terminal on B chain of human insulin:

B9-23p1: agcaggaagcctatcttccaggtta

B9-23p2: gcagaggggtaggctgggtagtggt

The amino acid sequence of B9-23 is: S H L V E A L Y L V C G ERG.

Proceed on by following Takara's RNA PCR Kit instruction; use Oligo (dT) as downstream primer for reverse transcription. Reaction condition: 42°C for 30 minutes, 99°C for 5 minutes, and 5°C for 5 minutes. Design specific primers with gene sequence for PCR reaction. Amplification parameters: 94°C for 2 minutes; 94°C for 30 seconds, 55°C for 30 seconds, and 72°C for 70 seconds, 35 cycles; 72°C for 10 minutes, take the reaction products for 1% agarose gel electrophoresis analysis. It is shown that the RT-PCR product of human insulin is a band of about 750bp, and the RT-PCR product of B9-23 is a band of about 250bp.

Recycle the PCR products by following the instruction of Takara Company's PCR Fragment Recovery Kit. The recycled cDNA fragment and pMD 18-T vector (purchased from Takara Company) is left overnight at 16°C under the effect of $T_4$ DNA ligase, and then make the human insulin's cDNA or B9-23 coding sequence connect to the vector of pMD 18-T. Connecting products should be transfered to competent cells. For the preparation and transfer of competent cells and extraction of plasmids, refer to reference (Sambrook J, Fritsch EF, Maniatis T. Molecular Cloning: A Laboratory Manual, (2nd ed). New York: Cold Spring Harbor Laboratory Press, 1989. 19-56). The extracted plasmids are identified by double enzyme digestion for recombinant plasmids with EcoR I and Hind III. After the enzyme digestion reaction, agarose gel electrophoresis analysis is done, and massive extraction of plasmids is done by correct cloning. In order to identify the cloned cDNA sequence, recombinant plasmids are purified. Use BcaBEST primer RV-M and BcaBEST primer M13-47 to do bi-DNA-sequence analysis with a PE377 full-automatic sequencer. The sequence obtained is analyzed by PE Company's SeqEd v1.0.3 software. Plasmids are named pMD-insulin, when they are the plasmids of human insulin cDNA gene, from the 56th to 388th DNA from the 5th terminal, identified by enzyme digestion and sequencing to contain nucleotide sequence GenBank Accession Number AY899304. Plasmids are named pMD-B9-23, when they are the plasmids of cDNA gene of B9-23, which are from the 152nd to 196th DNA from the 5th terminal, identified to contain nucleotide sequence GenBank Accession Number AY899304.

Cut off the human insulin genetic fragment and B9-23 fragment respectively from pMD-insulin and pMD-B9-23 with EcoR I and Xho. Connect the human insulin genetic fragment to its enzyme counterpart digested eukaryotic expression vector pVAX1 (from Invitrogen Company), and connect B9-23 fragment to its enzyme counterpart digested eukaryotic expression vector pcDNA3.0 (from Invitrogen Company). Do double enzyme digestion and sequencing of recombinant plasmids with EcoR I and Xho I. Plasmids are named pMD-insulin, when they are the plasmids of human insulin cDNA gene, from the 56th to 388th DNA from the 5th terminal, identified by enzyme digestion and sequencing to contain nucleotide sequence GenBank Accession Number AY899304. Plasmids are named pMD-B9-23, when they are the plasmids of cDNA gene of B9-23, which are from the 152$^{nd}$ to 196$^{th}$ DNA from the 5$^{th}$ terminal, identified to contain nucleotide sequence GenBank Accession Number AY899304.

Results of enzyme digestion of pVAX-insulin with EcoR I and Xho I are shown in Fig. 1a. 0.7% agarose gel electrophoresis analysis suggests that the product of pVAX-insulin after enzyme digestion with EcoR I and Xho I is an approximately 750bp human insulin cDNA fragment. In Fig. 1a, 1:DNA standard molecular weight (10000bp, 5000bp, 2500bp, 1000bp, and 250bp, purchased from Takara Company); 2, 3: enzyme digestion of recombinant plasmid pVAX-insulin with EcoR I and Xho I.

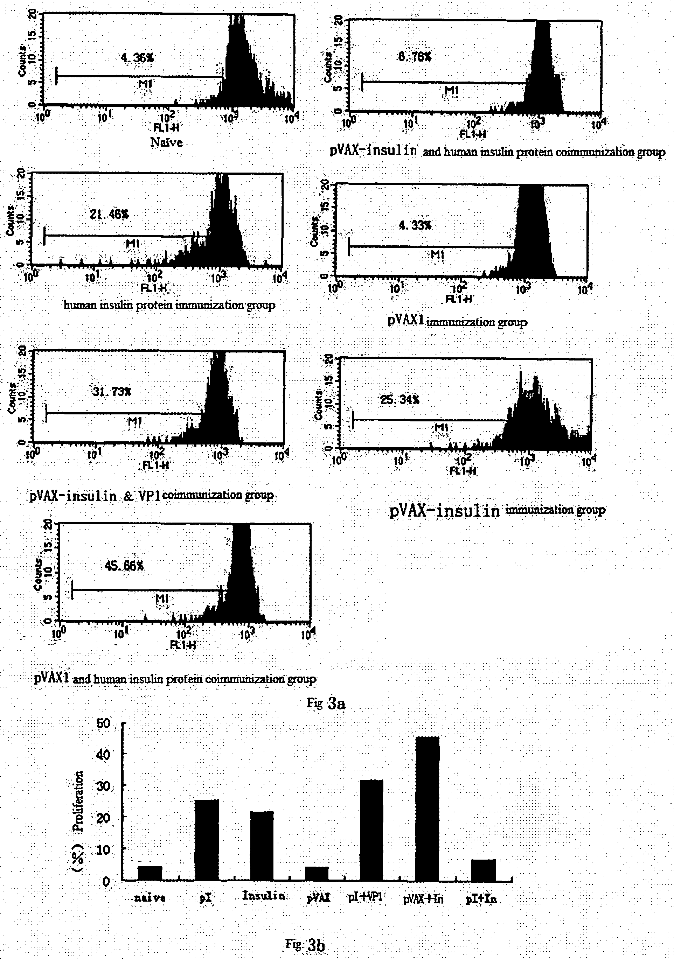

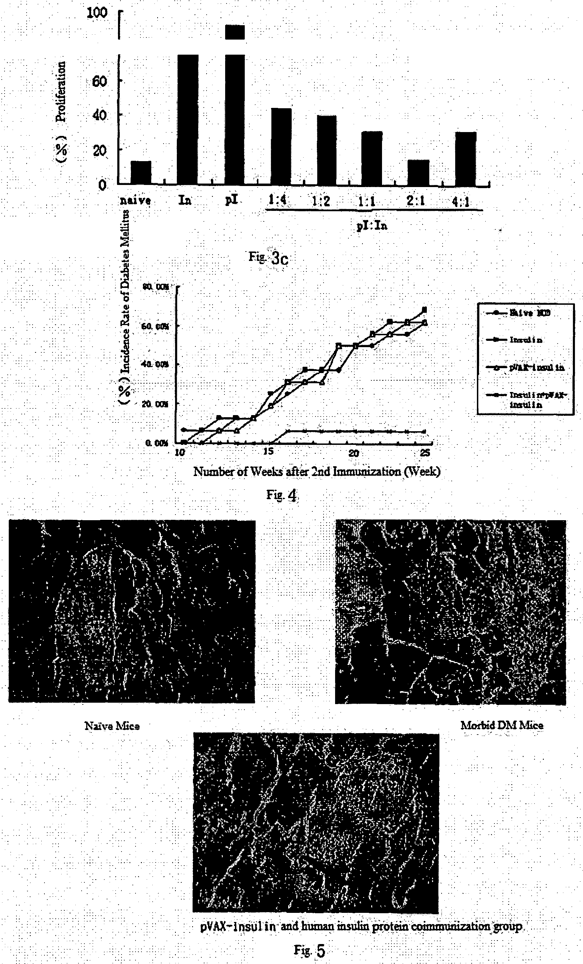

Results of enzyme digestion of pcDB9-23 vector with EcoR I and Xho I are shown in Fig. 1b. 0.7% agarose gel electrophoresis analysis suggests that the product of pcDB9-23 after enzyme digestion with EcoR I and Xho I is approximately 250bp B9-23 cDNA genetic fragment. In Fig. 1b, 3 is DNA standard molecular weight (2000bp, 1000bp, 750bp, 500bp, 250bp, and 100bp, purchased from Takara Company); 2: enzyme digestion of recombinant plasmid pCDB9-23 with EcoR I and Xho I.

By following lipofectamine product instruction and using lipofection, use pVAX-insulin to transfect BHK21 cells (from ATCC Company, USA). After 48 hours from the transfection, collect the cells and extract the complete RNA and examine the target genes' expressions by the RT-PCR approach, with mInsulinp1 and mInsulinp2 as primers. The results are as shown in Fig. 1c, indicating 750bp human insulin cDNA fragment in the transfected cells, which suggests effective external expression of pVAX-insulin at the mRNA level. 1:standard molecular weight (10000bp, 5000bp, 2500bp, 1000bp, and 250bp, purchased from Takara Company); 2, 3: pVAX-insulin transfected cell insulin's expression; 4: untransfected cells for contrast.

Embodiment 2: effect test of vaccines preventing and/or treating type I diabetes mellitus

Balb/c mice and NOD mice for separate immunization experiments. Intramuscular injection. 3 Balb/c mice in each group. 16 NOD mice in each group.

Experiment with Balb/c mice

1. Cellular immune response of Balb/c mice

9 Balb/c mice are divided into 3 groups of 3 each. Each mouse from group 1 (pcDB9-23 immune group) gets immunized with 100μl of 0.9% NaCl aqueous solution containing 100μg of pcDB9-23. Each mouse from group 2 (B9-23 immune group) gets immunized with 100μl of 0.9% NaCl aqueous solution containing 100μg of B9-23. Each mouse from group 3 (pcDB9-23 and B9-23 coimmune group) gets immunized with 100μl of 0.9% NaCl aqueous solution containing 100μg of pcDB9-23 and 100μg of B9-23. After 14 days from the first immunization, the same dosage is applied again for enhanced immunization, and on the seventh day after the second immunization, a T cell proliferation experiment is done with the following procedures.

Use the T cell proliferation test, CFSE staining, and flow cytometry examination to reflect T lymph cells' proliferation specific to particular antigens. The organism's lymph cells receive antigen (among other things) specific or non-specific simulation, which leads to activation of cells, synthesis of cytokines, cytokines receptor expression, and proliferation of activated cells. Proliferative reaction of cells can reflect the functional status of the cells, to a certain degree.

Here are the specific procedures: 1. Kill the mice by dislocating their joints. Soak the mice in 70% ethyl alcohol for 15 minutes; 2. on the clean bench for 20 minutes of advanced sterilization by ultraviolet lamp, under aseptic conditions, take the mice spleens into a cell culture dish in which 2ml of RPMI1640 culture solution has been added in advance; 3. Cool down brass wire mesh after ignition, put it into a plate, and grind the mice spleens with sterilized syringe to prepare cell suspension. Filter the suspension into a 13ml cell centrifuge tube; 4. seal the centrifuge tube with sealing film, centrifuge for 2000 revolutions and for 10 minutes; 6. discard the upper layer of culture solution, add 3-4ml RPMI1640 (including 2% fetal calf serum) culture medium suspension cells; 7. filter the cells slowly with glass wool at 37°C and make sure the cells fully integrate with the glass wool to remove B cells; 8. Count the cells with blood cell counting board; 9. wash away culture medium with PBS, and finally 1ml of PBS solution to suspend 2 X 10$^7$ cells; 10. Add 3uM of CFSE stock solution until the final concentration is 1.5uM, shake gently for 8 minutes at room temperature; 11. Add equal volume of fetal calf serum to terminate the reaction. Place the cells into water bath for 10 minutes, then after centrifugation at 2000rpm for 5 minutes, discard the supernatant and suspension cells, and wash cells with PBS solution (1ml/10$^6$ cells), centrifuge and then discard the supernatant. Triplicate this procedure; 12. Divide each group of cell suspension into 4 shares and add them into a 96-well culture plate. In one of the four shares of suspension, add 100μl Con A (mitogen) until the final concentration is 5μg/ml, in a second share of suspension add corresponding specific antigen (B9-23) as a stimulator until the final concentration is 5μg/ml, add no stimulator in the third share of suspension, and add 100μl of BSA as a unrelated antigen into the fourth share of suspension until the final concentration is 2μg/ml.

Meanwhile, prepare cells without any stimulator and without CFSE staining for contrast; 13. put the cells into cell incubator, 37°C, 5% $CO_2$ cultivation, and examine cell proliferation status respectively at the 48th and 96th hour.

Results of examination at the 48th and 96th hour show that the T cell proliferation level of pcDB9-23 and B9-23 coimmune group (proliferation at 0.08%) is significantly lower than that of pcDB9-23 immune group (proliferation at 22.32%) and the B9-23 immune group (proliferation at 8.94%), and prove that immunosuppression happens to pcDB9-23 and B9-23 coimmune group. Fig. 2a and Fig. 2b indicate the proliferation level with the percentages. The greater the values, the higher the level of proliferation. M1 represents the percentage of cell proliferation.

2. Specificity of cellular immune response

This procedure is to further examine whether immunosuppression has specificity, meaning that it only performs suppression of insulin antigen's protein and DNA. Divide 21 Balb/c mice into 7 groups of 3 each. Immunize each mouse in group 1 (negative control group, i.e. naive) with 100μl of 0.9% NaCl aqueous solution, immunize each mouse in group 2 (pVAX-insulin immune group) with 100μl of 0.9% NaCl aqueous solution containing 100μg of pVAX-insulin, immunize each mouse in group 3 (human insulin protein immune group) with 100μl of 0.9% NaCl aqueous solution containing 100μg of human insulin protein, immunize each mouse in group 4 (pVAX1 immune group) with 100μl of 0.9% NaCl aqueous solution containing 100μg of pVAX1, immunize each mouse in group 5 (pVAX-insulin and VP1 coimmune group) with 100μl of 0.9% NaCl aqueous solution containing 100μg of pVAX-insulin and 100μg of Foot-and-mouth disease virus (FMDV) "VP1" (as per the method of preparation described in the reference: Jin Huali, Zhang Fuchun, Shan Wenjuan, Zhang Ailian, Li Yijie, and Wang Bin, Expression of FMDV VP1 Protein in Pichia Pastoris and Its Immunological Activity in Mice. Cellular and Molecular Immunology, 2004, 20 (5) 513-516). Group 5 is taken as a control group. Immunize each mouse in group 6 (pVAX1 and human insulin protein coimmune group) with 100μl of 0.9% NaCl aqueous solution containing 100μg of pVAX1 and 100μg of human insulin protein. After 14 days from the first immunization, use equal dosage to immunize again for enhancement, and do a T cell proliferation experiment following the aforementioned step 1 on the seventh day after the second immunization. The results after 48 hours of stimulation are as illustrated in Fig. 3a and Fig. 3b, showing that the immunosuppression occurs only when the insulin protein is mixed with the corresponding DNA (as in pVAX-insulin and human insulin protein coimmune group). With other combination groups and control groups, T cells activity does not show significant decrease. This suggests that coimmunization is the specific relationship between protein and DNA of the same antigen. Fig. 3a indicates the proliferation level with the percentages. The greater the values, the higher the level of proliferation. M1 represents the percentage of cell proliferation. In Fig. 3b, pI represents the pVAX-insulin group, Insulin represents the human insulin protein immune group, pVAX represents the pVAX1 immune group, pI+VP1 represents the pVAX-insulin and VP1 coimmune group, pVAX+In represents the pVAX-insulin and human insulin protein coimmune group, and naive represents the negative control group.

3. Experiment of dosage relationship in cellular immune response

This experiment is to explore the dosage relationship between protein and DNA upon the optimum effectiveness of suppression. Divide 24 Balb/c mice into 8 groups of 3 each. Immunize each mouse in group 1 (naive) with 100μl of 0.9% NaCl aqueous solution, immunize each mouse in group 2 (pVAX-insulin immune group) with 100μl of 0.9% NaCl aqueous solution containing 100μg of pVAX-insulin, immunize each mouse in group 3 (human insulin protein immune group) with 100μl of 0.9% NaCl aqueous solution containing 100μg of human insulin protein, immunize each mouse in group 4 (pVAX-insulin and human insulin protein 1:4 coimmune group) with 100μl of 0.9% NaCl aqueous solution containing 25μg of pVAX-insulin and 100μg human insulin protein, immunize each mouse in group 5 (pVAX-insulin and human insulin protein 1:2 coimmune group) with 100μl of 0.9% NaCl aqueous solution containing 50μg of pVAX-insulin and 100μg of human insulin protein, immunize each mouse in group 6 (pVAX-insulin and human insulin protein 1:1 coimmune group) with 100μl of 0.9% NaCl aqueous solution containing 100μg of pVAX-insulin and 100μg of human insulin protein, immunize each mouse in group 7 (pVAX-insulin and human insulin protein 2:1 coimmune group) with 100μl of 0.9% NaCl aqueous solution containing 200μg of pVAX-insulin and 100μg of human insulin protein, and immunize each mouse in group 8 (pVAX-insulin and human insulin protein 4:1 coimmune group) with 100μl of 0.9% NaCl aqueous solution containing 400μg of pVAX-insulin and 100μg of human insulin protein. After 14 days from the first immunization, use an equal dosage to immunize again for enhancement, and do the T cell proliferation experiment following the aforementioned step 1 on the seventh day after the second immunization. The results after 48 hours of stimulation are as illustrated in Fig. 3c. Suppression is the most significant when the ratio of plasmid to protein is 2:1. In Fig. 3c, naive represents negative control group, pI represents pVAX-insulin immune group, In represents human insulin immune group. The ratios 1:4, 1:2, 1:1, 2:1, and 4:1 respectively represent pVAX-insulin and human insulin protein 1:4 coimmune group, pVAX-insulin and human insulin protein 1:2 coimmune group, pVAX-insulin and human insulin protein 1:1 coimmune group, pVAX-insulin and human insulin protein 2:1 coimmune group, pVAX-insulin and human insulin protein 1:4 coimmune group, and pVAX-insulin and human insulin protein 4:1 coimmune group.

4. Coimmunization prevention experiment with NOD mice

Divide 64 female NOD mice into 4 groups of 16 each. Immunize each mouse in group 1 (naive) with 100$\mu$l of 0.9% NaCl aqueous solution, immunize each mouse in group 2 (pVAX-insulin immune group) with 100$\mu$l of 0.9% NaCl aqueous solution containing with 100$\mu$g of pVAX-insulin, immunize each mouse in group 3 (human insulin protein immune group) with 100$\mu$l of 0.9% NaCl aqueous solution containing 100$\mu$g of human insulin protein, and immunize each mouse in group 4 (pVAX-insulin and human insulin protein coimmune group) with 100$\mu$l of 0.9% NaCl aqueous solution containing 100$\mu$g of pVAX-insulin and 100$\mu$g of human insulin protein. After 14 days from the first immunization, use equal dosages to immunize again for enhancement, and examine and record the changes of the mice' blood glucose levels with a glucose meter (purchased from Beijing Yicheng Company) on a weekly basis. Any of the NOD mice whose glucose level exceeds 200mg/dl for two successive weeks are deemed to have diabetes mellitus.

As Fig. 4 shows, prevalence of each group of NOD mice is calculated. Under normal conditions, the prevalence of diabetes mellitus in the female NOD mice is around 60%. In pVAX-insulin and human insulin protein coimmune group, the prevalence in the NOD mice is significantly lower, with only one mouse getting the disease, and occurrence of the disease is significantly delayed. This suggests that coimmunization with pVAX-insulin and human insulin protein can effectively prevent type I diabetes mellitus. In Fig. 4, Naive NOD represents mice in the negative control group, Insulin represents mice in the human insulin protein immune group, pVAX-insulin represents mice in the pVAX-insulin immune group, and Insulin+pVAX-insulin represents mice in the pVAX-insulin and human insulin coimmune group.

Pancreatic tissues of the mice in the naive group, the mice that developed diabetes mellitus, and the mice in pVAX-insulin and human insulin protein coimmune group are taken for sectioning and HE staining. The results also demonstrate that infiltration of lymph cells in the pancreatic islet tissue of the pVAX-insulin and human insulin protein coimmune group is less severe than that of the other groups of diseased mice, and similar to that of the normal NOD mice without occurrence of the disease (i.e. the naive mice) (see Fig. 5).

The experiment proves that coimmunization with insulin DNA and protein can effectively prevent type I diabetes mellitus. The immunosuppressive nature of the coimmunization is repeatedly verified in the experiment. Especially for NOD mice, it is able to prevent type I diabetes mellitus.

In summary, the experiment proves a new method to prevent type I diabetes mellitus. Future exploration of relevant mechanisms might help develop a new method to treat autoimmune diseases in human and animals.

Embodiment 3: treating/preventing autoimmune oophoritis by coimmunization

Abstract: Autoimmune ovarian disease (AOD) is one of the causes of predisposion to premature ovarian failure (POF) in humans. Zona pellucida protein (ZP3) is a protein autoantigen of AOD.

With an AOD mice model induced by MZP3 protein and CFA, we examine a more practical technology to treat oophoritis. Based on the results that we have previously observed, which point to the fact that coimmunization with matching DNA and protein can induce immunosuppression of T cells; we improve AOP by means of coimmunization. Results show that coimmunization with MZP3 DNA and protein improves AOD, inhibits the reaction of antigen-specific T cells, and reduces the expression levels of inflammatory factors IL-12 and INF-$\gamma$. These effects might have been achieved as coimmunization induces forth a group of specific regulatory T cells. This group of regulatory T cells is characterized with CD4[+]/CD25[+]/FoxP3[+] and expression of IL-10. We are the first to report the treatment of autoimmune diseases through coimmunization with DNA and protein.

For centuries and also in recent years, studies have found out there is an intimate and complicated relationship between the immune system and the reproductive system. [1,2] On the ground of this relationship, a new class of immunocontraceptive technology is developed. Based on molecular biotechnology including gene recombination, this technology takes a key factor in reproductive process as the vaccine of target antigen, disables intrinsic target antigens by making use of the immune system's response to extrinsic target antigens, obstructs the fertility of overbreeding animals, and disturbs the normal reproductive process of overbreeding animals, so as to maintain a dynamic balance among biological species and with their environment. [3] The egg membranes of mice are all covered by a layer of zona pellucida. Zona pellucida is an acidophilic film containing glycoprotein formed by secretion of oocytes and primary oocytes at the early stage of the primary oocyte maturation process The zona pellucida consists of ZP1, ZP2, and ZP3. [4] Zona pellucida plays two main roles in the process of fertilization: It is responsible for sperm binding, and it initiates the acrosome reaction to let the sperms into the eggs. [5] As the major glycoprotein in zona pellucida, ZP3 is the primary receptor of sperm, is even more important in the process of the sperm-egg binding, and has been considered as ideal for immunocontraceptive vaccines. [6]

Nevertheless, preparation of an immunocontraceptive vaccine is often found to be accompanied by occurrence of oophoritis. Dunbar et al. [7] uses natural swine ZP glycoprotein and deglycosylated ZP protein for active immunization with female individuals and that leads to interruption of fertilization. However, studies have found that some of the immunized animals have transient changes in their ovulation cycles, hormone level, and development of ovarian follicles, while the others of the immunized animals have irreversible changes in these particular aspects.

The normal age for women's menopause is generally at the age of 50, and loss of follicular function before the age of 40 can be considered premature ovarian failure (POF). Prevalence of this disease in women is usually 1% to 2%, with some especially serious cases occurring in teenagers [8]. Autoimmune ovarian disease (AOD) is thought to be one of the major causes of predisposition to POF[9-11]; Rhim et al. establish a new animal model of experiment with mice by taking a piece of small peptide (330 to 342) after injection with MZP3 on the sole and underneath the skin. They use the model for study of ovarian autoimmune diseases, and this model, to a certain extent, can simulate women's premature ovarian failure (POF)[12]. This autoimmune disease is mediated by CD4[+] T cells, and Lou et al. discovered that the production of autoantibodies can change the distribution of T cell-mediated inflammatory reaction, and can result in loss of functional units of target organs[13].

As the controller of an organism's immune system, regulatory T cells can be in charge of controlling the immune response of the organism to its antigen, and thus protect the organism against autoimmune diseases. Natural regulatory T cells are normally CD4[+] CD25[+] double-positive T cells. This kind if cells can express a function transcription factor, namely, PoxP3, which is the important cellular component of a normal immune system [14] . Existence of such cells enables researchers use antigen-specific regulatory T cells to treat autoimmune diseases and allograft rejection[11-17]. Samy et al. use antigen dependent CD4[+]CD25[+] double-positive T cells to control the occurrence of oophoritis, which is an autoimmune disease[18]. Jin et al. discovered that coimmunization with protein and DNA can inhibit antigen-specific cellular response [19], and that this suppressive function might be related to the antigen-specific regulatory T cells.. Therefore, with mice as the animal model, this paper explores methods to treat autoimmune oophoritis and respective mechanisms based on the method of inhibiting cellular response by coimmunization with protein and DNA.

1 Materials and procedures

1.1 Materials and reagents

pMD18-T sequencing vector is purchased from Takara Company, and Escherichia coli DH5αbacterial strain is the conserved strain from our laboratory. RNA extraction kit, PCR product recycling kit, DNA marker, restriction enzyme, exTaq enzyme, and RT-PCT and PCR primers are all purchased from Takara Company. Sequencing kit is purchased from PE (USA) Company, and the other reagents are analytical reagents.

1.2 Cloning of MZP3 genes

Take ovaries from sexually mature mice to be fully ground in Trizo. Follow the instructions to proceed with extraction of the complete RNA. Design primers according to the published gene sequence (GenBank number: BC103585). The primers' sequences are:

MZP3 P1: AATGAATTGATGAATTCCCAGACTCTGTGGC

MZP3 P2: TTACTCGAGTTAAGTCCAGCCTTCCACAGTCT

The amplified fragment is from the 63rd to 1143rd bases in the nucleic acid sequence of MZP3.

Proceed on by following Takara's RNA PCR Kit instructions, using Oligo (dT) as downstream primer for reverse transcription. Reaction conditions: 42°C for 30 minutes, 99°C for 5 minutes, and 5°C for 5 minutes. Design specific primers with MZP3 gene sequence for PCR reaction. Amplification parameters: 94°C for 2 minutes; 94°C for 30 seconds, 55°C for 30 seconds, and 72°C for 70 seconds, 35 cycles; 72°C for 10 minutes, take the reaction products for 1% agarose gel electrophoresis analysis. Follow Takara's PCR Fragment Kit instruction to recycle PCR products. The recycled MZP3 cDNA fragment and pMD 18-T vector is left overnight at 16°C under the effect of $T_4$ DNA ligase, and make MZP3 cDNA's coding sequence connected to the vector of pMD 18-T. Connecting products are transfered to competent cells. For the preparation and transfer of competent cells and extraction of plasmids, refer to the reference (Sambrook J et al. 1989)[20]. Extract a small quantity of plasmid DNA as per related reference (Sambrook J et al. 1989), identify the extracted plasmids by BamH I and Hind III double enzyme digestion for recombinant plasmids. After the enzyme digestion reaction, agarose gel electrophoresis analysis is done, and massive extraction of plasmids is achieved by correct cloning. In order to identify the cloned cDNA sequence, recombinant plasmids are purified. Use BcaBEST primer RV-M and BcaBEST primer M13-47 to do bi-DNA-sequence analysis with a PE377 fully automatic sequencer. The obtained sequence is analyzed by PE Company's SeqEd v1.0.3 software.

1.3 Construction of eukaryon expression vector and transient expression of recombinant plasmids in BHK21 cells

Do EcoR I and Xho I double enzyme digestion of the above, connect them to the corresponding enzyme digested eukaryon expression vector pcDNA3, and name the vector as pcDmzp3. Conduct the eukaryon transfection experiment with the recombinant plasmids that are correct as per sequence identification. For the specific procedures of transfection, refer to lipofectamine product instructions. Transfect BHK21 cells with pcDmzp3 by means of lipofection. See the instructions for specific procedures. Forty-eight hours after transfection, collect the cells, extract the complete RNA and examine the expression of the target gene by means of RT-PCR with MZP2 P1 and MZP3 P2 as primers.

1.4 Construction of the eukaryon expression vector, protein expression, and protein purification

Do EcoR I and Xho I double enzyme digestion of the recombinant plasmids pMD18-T/MZP3, connect to the corresponding enzyme digested eukaryon expression vector pGEX-4T-1, and proceed with the protein expression of the recombinant plasmids that are correct as per sequence identification. Select individual bacterial colony to inoculate into fresh LB (containing Amp$^+$ 50mg/L) culture medium and leave it overnight at 37°C. On the following day, inoculate into fresh LB (containing Amp$^+$ 50mg/L) culture medium at 1% of the concentration. When A600 reaches 0.6-0.8, induced by 1.0mmol/L IPTG, express it for 5 hours at 37°C. Take 1ml of the bacterial solution to centrifuge for collection of bacterial cells. Then wash it once with double distilled water, suspend it in 1xSDS liquid sample, and cook for 10 minutes in boiling water bath. Centrifuge for 2 minutes at 12000g. Take 15$\mu$l of supernatant for 100g/L SDS-PAGE, and stain with Coomassie brilliant blue.

Centrifugally collect the E.coli BL21 (DE3) bacterial cells after stimulus, wash with PBS, and then use 1/20 original cultivating volume of cell lysate L1 (100mmol/L Tris-HCl (pH 8.0), 1mmol/L EDTA, and 200mmol/L NaCl) to resuspend the bacterial precipitate. After 15 minutes of ice bath, ultrasonicate bacteria until the bacterial solution is no longer sticky. Centrifuge at 4°C, 12000g for 10 minutes and discard the supernatant. Collect the precipitate to obtain preliminarily prepared MZP3 protein inclusion bodies. Wash the preliminarily prepared protein inclusion bodies with L1 and 4M urea, warm it at 70°C for 10 minutes, and centrifuge at 4°C, 12000g for 5 minutes, repeatedly for 3 times. Use L3 (10mmol/L Tris-HCL, 1mol/L NaCl, 8M urea, 5 mmol/L $\beta$-Mercaptoethanol and 5mmol/L DTT, pH10) to dissolve the protein inclusion bodies. Warm it at 55°C for 10 minutes, and centrifuge at 4°C, 12000g for 5 minutes to remove the precipitate. Put the supernatant into a dialysis bag to dialyze for 8 hours in L2 containing 4M urea (10mmol/L Tris-HCl (pH 8.0) and 1mol/L NaCl), and then dialyze for another 8 hours in L2 containing 2M urea. Gradually thin down the concentration of the urea, until the urea is removed from the protein solution, and use Bradford to measure protein content.

1.5 Inducement of oophoritis model

Purchased from the institute of Laboratory Animal Science, Chinese Academy of Medical Sciences (CAMS), the C57BL/6 mice are female at the age of 6 to 8 weeks. Each of the mice is immunized on the sole and muscle with 100$\mu$l of fully emulsified 1:1 mixture of protein (concentration being 2mg/ml) and complete Freund's adjuvant (CFA). That is, each of these mice is immunized with 100$\mu$g of protein. The mice immunized with CFA only are taken for contrast. After 14 days, draw blood and collect serum, and examine corresponding antibody titer by indirect ELISA. Use purified GST-MZP3 fusion protein as the standard antigen, examine the specific antibody level of MZP3 protein in the mice serum by ELISA. Dilute the protein to 5$\mu$g/ml, immerse in a 96 well ELISA plate with 100$\mu$l/well and leave the plate overnight at 4°C. Discard the solution and wash the plate with PBST 3 times. Keep 5% skim milk powder-PBST sealed for 1 hour at 37°C. After washing the plate, fill in mice serum of different dilutions and incubate for 2 hours at 37°C. After washing the plate, add in 1:1000 HRP-IgG, 50$\mu$l/well, incubate at 37°C for 2 hours and then discard. Wash the plate, fill in 50$\mu$l/well TMB, and leave for color development reaction for 30 minutes at room temperature without light. Use 2mol/L sulphuric acid to terminate the reaction. Use ELISA reader to get the value of OD450/650. Histological assessment of ovarian diseases: Fix the ovaries in Bouin's fixing solution for 24 hours. Proceed with paraffin embedding, and then do a serial section (5$\mu$m) and HE (hematoxylin and eosin) staining. Ovarian pathology grading by severity: 1. Inflammation in interstitial area; 2 & 3. Inflammatory reaction at multiple additional sites, or granuloma between ovarian follicles or internal granuloma; and 4. Disappearance of ovarian follicles and atresia ovary. Proliferative response of antigen-specific lymph cells: 14 days after immunization, kill the mice and take out popliteal lymph node under aseptic conditions. Grind the lymph node, centrifuge at 2000rpm for 5 minutes, then discard the supernatant and resuspend cells with culture medium. Count with a blood counting chamber and adjust the cellular concentration to be 1 x 10$^7$/ml. Take 2 x 10$^7$ cells, centrifuge at 2000rpm for 5 minutes and then discard the supernatant. Use aseptic PBS to resuspend cells, add in 1.5$\mu$l of CFSE (1mmol/ml), and gently shake for 10 minutes at 37°C. Add in equal volumes of calf serum to terminate the response. Centrifuge at 2000rpm for 5 minutes, discard the supernatant, and wash with PBS three times. Finally use 1ml of culture medium to resuspend cells, fill 100$\mu$l of cells in each well, and use MZP3 (10$\mu$g/ml) protein to stimulate T cells proliferation. Take BSA (2$\mu$g/ml) as a non-specific antigen for contrast, and ConA (10$\mu$g/ml) for positive contrast.

Use flow cytometry to examine the status of proliferation. By means of intracellular staining, examine cytokines IL-12 and INF-$\gamma$: 14 days after immunization, execute the mice, take out their spleens under aseptic conditions, and grind the spleen tissues. Centrifuge at 2000rpm for 5 minutes and discard the supernatant. Use 1-2ml of red blood cell lysis buffer to treat the cells for 2-3 minutes, and add 6-12ml of RPMI 1640 culture medium containing 4% serum to terminate. Centrifuge at 2000rpm for 5 minutes and discard the supernatant. Resuspend cells with culture medium. After counting with the blood counting chamber, adjust the cellular concentration to be 2 x $10^6$/ml. Preparation of lymph node single cell suspension is the same as described above. Fill each well with be 2 x $10^6$ cells (100$\mu$l), use 10$\mu$g/ml of MZP3 protein to stimulate for 4-6 hours, add monensin to suppress for 1-2 hours. Then do intracellular staining and use flow cytometry to examine the expression status of cytokines. Here are the specific procedures: (1) Kill the mice after 21 days from immunization, prepare spleen single cell suspension, add in 2ml of red blood cell lysis buffer to dissolve red blood cells, wash once with washing liquid, centrifuge, resuspend the cells and count; (2) Seal anti-Fcg antibodies: From each group of mice, take 1 x 106 cells, add in appropriate amount of anti-Fcg antibodies (see instruction for the amount), and seal for 30 minutes; (3) add in 2-3ml of washing liquid, centrifuge at 2000rpm for 5 minutes, discard the supernatant, add appropriate amount of fluorescence labeled antibodies in each tube, mix well, and leave in darkness for reaction at 4°C for 30 minutes; (4) add in 2-3ml of washing liquid, centrifuge at 2000rpm for 5 minutes, discard the supernatant, add in 0.2ml of 4% paraformaldehyde and fix for 20 minutes at 4°C; (5) add in 2-3ml of washing liquid, centrifuge at 2000rpm for 5 minutes, discard the supernatant, add in 0.2ml of membrane rupturing agent to rupture the cells, mix well, leave at room temperature for 15 minutes in darkness for reaction; (6) add in 2-3ml of washing liquid, mix well, centrifuge at 2000rpm for 5 minutes, discard the supernatant, add appropriate amount of fluorescence labeled monoclonal antibodies in each tube (see instruction for the amount) and mark intracellular analytes; (7) do intracellular staining of antibodies, mix well with cells, and leave in darkness for reaction at 4°C for 30 minutes; (8) Add in 2-3ml of washing liquid, mix well, centrifuge at 500xg for 5 minutes, discard the supernatant, and add 300$\mu$l of washing liquid into each tube to resuspend the cells; (9) do flow cytometry analysis. Apply RT-PCR to examine cytokine IL-2: Follow the instructions of TRIZOL Reagent (Gibco), take $10^7$ cells from each group, use 1ml of TRIzol Reagent for lysing, and then add 200$\mu$l of chloroform and mix well. Centrifuge at 12000r/min for 15 minutes at 4°C, take the upper aqueous layer and transfer it to a new EP tube. Add 500$\mu$l of isopropyl alcohol, mix well and set still for 10 minutes at room temperature. Centrifuge at 12000r/min for 10 minutes at 4°C and then discard the supernatant. Wash RNA precipitate with 75% ethyl alcohol, and then dissolve it in 20$\mu$l of DEPC-treated ultrapure water, and store at -80°C. Take 1$\mu$l of RNA sample and add in 599$\mu$l ofultrapure water. Compare colors on an ultraviolet spectrophotometer, read the OD values of A260 and A280. Follow the instructions of the reagent kit to proceed with RT-PCR, and examine the amplified products with a 1% agarose gel electrophoresis analysis.

1.6 Treating oophoritis by coimmunization

Mix 2mg/ml protein with CFA at 1:1, fully emulsify the mixture, and then immunize each mouse with 100$\mu$l on the sole and muscle. After 14 days, do intramuscular immunization with pcDmzp3+MZP3, 100$\mu$g each. Immunize mice with pcDNA3, pcDNA3+MZP3, pcDmzp3, pcDmzp3+OVA, and MZP3 as control groups. After another 14 days, enhance immunization. One week after the enhanced immunization, take out the mouse spleen cells under aseptic conditions, do a T cell amplification experiment by means of MTT, use MZP3 protein antigen to stimulate T cells' proliferation, take BSA as non-specific antigen for contrast, and ConA as positive contrast. Here are the specific procedures: Use MTT to examine the proliferation activity of mice T cells in vitro.

(1) Take out the spleens: 7 days after the enhanced immunization, kill the mice and take out the spleens under aseptic conditions; (2) prepare single cell suspension: grind the spleen tissues, centrifuge at 2000rpm for 5 minutes and discard the supernatant, use 1-2ml of red blood cell lysis buffer to treat the cells for 2-3 minutes, and add 6-12ml of RPMI 1640 culture medium containing 4% serum to terminate; centrifuge at 20000rpm for 5 minutes and discard the supernatant, and resuspend the cells with culture medium.

(3) Count the cells: After counting the cells with the blood cell counting chamber, adjust the cellular concentration to be 3 x $10^6$/ml; (4) add in cell plates: 100$\mu$l of cells into each well, respectively add in ConA (final concentration being 10$\mu$g/ml), MZP3 protein (final concentration being 10$\mu$g/ml), and BSA (non-specific antigen, final concentration being 2$\mu$g/ml) to stimulate for 48-72 hours; (5) develop color: fill each well with 20$\mu$l of MTT solution, 37°C, 5% $CO_2$, cultivate for 3-4 hours. Centrifuge at 2000rpm for 5 minutes, discard the supernatant, add 100$\mu$l of dimethyl sulfoxide (DMSO) into each well, and shake gently for 20-30 minutes at 37°C; (6) read the values: use ELISA reader (Magellan, Tecan Austria GmbH) to get the OD value of 595nm; and (7) calculate the results: Stimulation index (SI):

$$SI = (\text{OD value of each stimulated well} - \text{OD value of culture medium}) / (\text{OD value of un-stimulated well} - \text{OD value of culture medium}).$$

Lymph cells amplification experiment: Under aseptic conditions, take out the mice's popliteal lymph node, grind the lymph node, centrifuge at 2000rpm for 5 minutes and discard the supernatant. Use culture medium to resuspend cells and prepare single cell suspension. Adjust the cellular concentration to be $3 \times 10^6$/ml. The remaining procedures are the same as described above.

Examination of cytokines IL-2, IL-12, INF-$\gamma$, IL-10, FoxP3, and CD25 as well as oophoritis assessment are the same as above.

2. Results

2.1 Gene cloning and sequence analysis

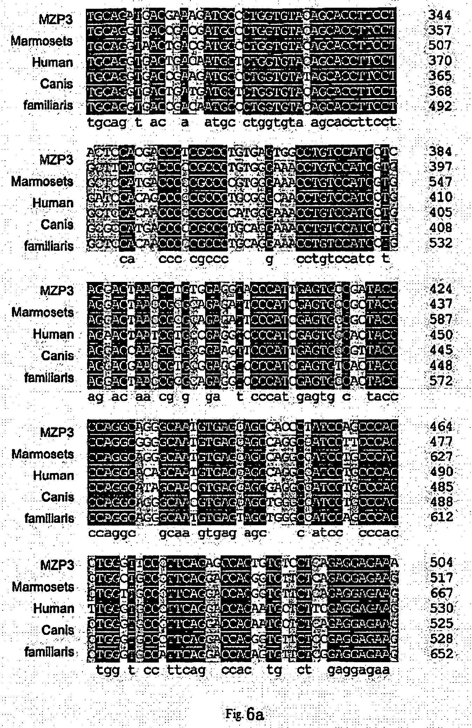

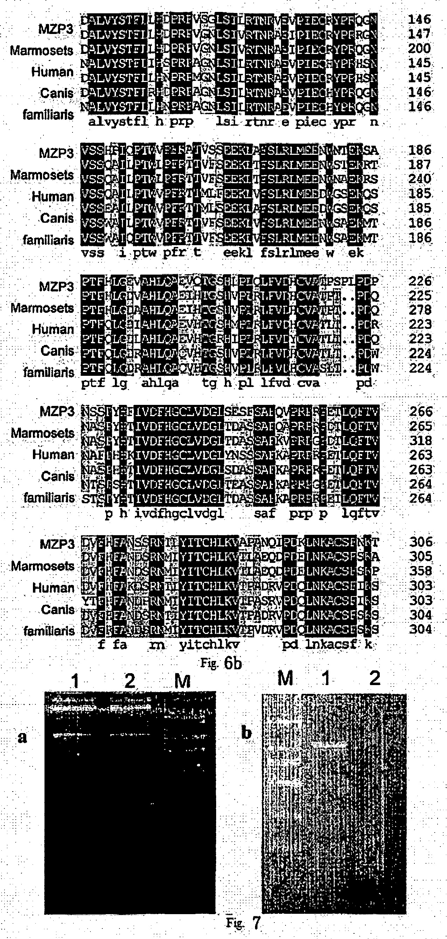

Use extracted total RNA and take oligodeoxythymine as primer for reverse transcription to get single chain cDNA, and use designed PCR primer for amplification to get MZP3 genetic fragment. Take RT-PCR products to do 1% agarose gel electrophoresis analysis, and a band of about 1200bp is seen, which is consistent with our expectations. Connect RT-PCR products and cloning vector pMD18-T, construct recombinant plasmid pMD18-T/MZP3. The recombinant plasmid goes through BamH I and Hind III enzyme digestion, and an approximately 1200bp DNA fragment is obtained with the same size junction fragment as we expected. For pMD18-T/MZP3 recombinant plasmid, double-direction sequencing is done with BcaBESTprimer RV2M and BcaBEST primer M13247, and the result of sequencing shows 99% homology to the published sequence. By analyzing the MZP3 genes of Marmosets, human, Canis familiars, felis catus, Sus scrofa, and Bos taurus, we have found 73% homology (see Fig. 6a), and 71% homology in their amino acid sequence (see Fig. 6b).

2.2 Construction of eukaryon expression vector and transient expression of recombinant plasmid in BHK21 cells

Do double enzyme digestion of recombinant plasmid pMD18-T/MZP3 and eukaryon expression vector pcDNA3 with EcoR I and Xho I. Recycle, purify, and then connect. Clone MZP3 genes into eukaryon expression vector pcDNA3 to obtain recombinant vector pcDmzp3. Do double enzyme digestion of recombinant plasmid with EcoR I and Xho I. Through 0.7% agarose gel electrophoresis analysis, a band is seen at about 1200bp (see a in Fig. 7) indicating successful construction of recombinant plasmid pcDmzp3. Use purified and quantized plasmid for lipofection of prosperous BHK21 cells, and collect cells after 72 hours. By means of RT-PCR, with MZP3p1 and MZP3p2 as primers, examine the expression status of target genes. Corresponding bands are found at 1200bp, as shown by b in Fig. 7. This means effective in-vitro expression of mRNA level of recombinant vector.

2.3 Construction of eukaryon expression vector, protein expression and purification

Do double enzyme digestion of recombinant plasmid pMD18-T/MZP3 and eukaryon expression vector pGEX4T-1 with EcoR I and Xho I. Recycle, purify, and then connect. Clone MZP3 genes into eukaryon expression vector pGEX4T-1 to obtain recombinant vector pGEX4T-1/MZP3. Do double enzyme digestion of recombinant plasmid with EcoR I and Xho I. Through 0.7% agarose gel electrophoresis analysis, a band is seen at about 1200bp (see a in Fig. 8) indicating successful construction of recombinant plasmid pGEX4T-1/MZP3. Use purified and quantized plasmid for transformation of competent cells E. coli BL21 (DE3), and cultivate the constructed E. coli BL21 (DE3)/MZP3 in fresh LB culture medium, and collect the transformed bacteria after IPTG inducement. Take its complete cell protein to proceed with SDS-PAGE. Results of SDS-PAGE show that the inducement expressed protein band exists in the precipitate of the cell lysate, and that suggests the protein is expressed as inclusion bodies. By purifying inclusion bodies, we dissolve, wash, and renature the protein to get highly pure protein (see c in Fig. 8).

2.4 Inducement of autoimmune oophoritis

Adjust the concentration of the aforementioned purified MZP3 protein to be 2mg/ml and fully emulsify with equal volume of CFA, and then immunize 8-10 weeks old female C57BL/6 mice by injection on the sole and muscle, 100$\mu$l for each mouse. Mice injected with 100$\mu$l of CFA are taken as the adjuvant control group, and mice injected with 100$\mu$l of PBS are taken as the negative control group (naive). After 14 days, kill the mice and take out the ovaries to fix for 24 hours in Bouin's fixing solution, and then embedded with paraffin. Do serial sectioning and HE (hematoxylin and eosin) staining. Results show that seven out of eight mice immunized with MZP3 protein and CFA have got oophoritis to different degrees, and the prevalence rate is 87.5% (see 9a). Ovarian stroma, growing and mature ovarian follicles are found with inflammatory response. The follicles are infiltrated by inflamed cells and this represents a severe loss of ovarian eggs (see Fig. 9b). By contrast, only mice injected with CFA remained free of oophoritis.

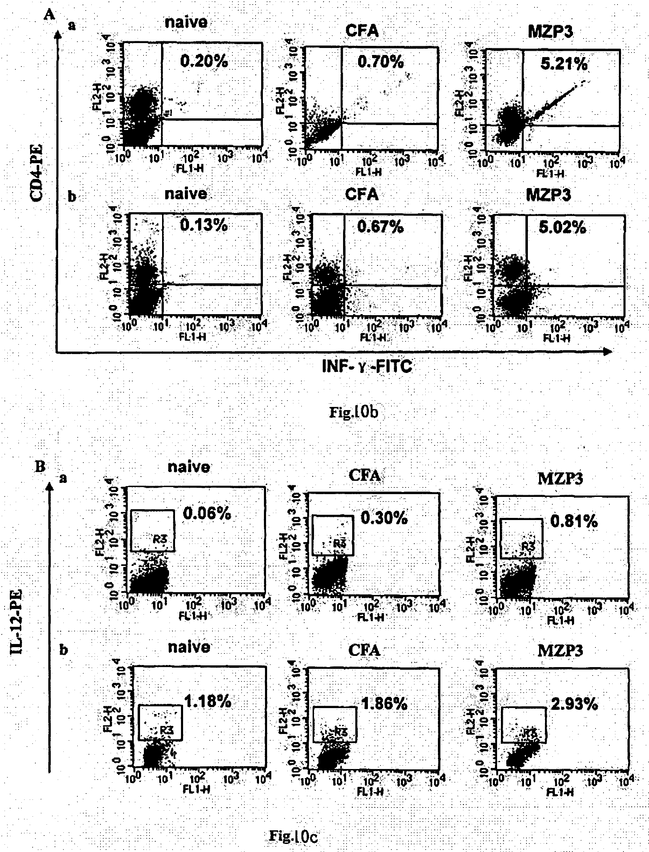

We examined anti-MZP3 antibody of collected serum by means of ELISA. After 14 days, compared with control group, MZP3 antibodies appeared in serum, and antibody titer reached 25600 (see Fig. 9c). Immunization with MZP3 protein and CFA resulted in strong T cells response (see Fig. 9d), and expression levels of cytokine IL-2 (see Fig. 10a), INF-γ (see Fig. 10b) and IL-12 (see Fig. 10c) improved. This means Th1 immunization response is activated. These results show that oophoritis was successfully induced and enabled evaluation of the treatment effectiveness of the method.

2.5 Coimmunization with MZP3 DNA and protein inhibits occurrence of AOD

By constructing an AOD model, we have examined the ability of coimmunization strategy in treating oophoritis. C57BL/6 mice are first immunized with MZP3 protein and CFA. After 14 days, mice receive intramuscular immunization with MZP3 protein and plasmid pcDmzp3, and an enhanced immunization after another two weeks. Check occurrence of oophoritis after 7 days from the second immunization. Results show that AOD suppression has only happened to those mice immunized with coimmunization plasmid pcDmzp3 and MZP3 protein (named as pcDmzp3+MZP3). In order to rule out the influence of non-specific factors, such as skeleton sequence or unrelated proteins, we have also established immunized blank plasmid pcDNA3 and MZP3 protein coimmunization (named as pcDNA3+MZP3) as well as plasmid pcDmzp3 and OVA protein coimmunization (named as pcDmzp3+OVA) as control groups. Results show that the control groups do not have AOD suppression, and that means the occurrence of AOD can only be suppressed within the mice in pcDmzp3+MZP3 coimmunization group, and thus suggests that the suppression is antigen-specific. Histological analysis has also revealed that the ovaries of mice immunized with pcDNA3+MZP3 or pcDmzp3+OVA have had inflammatory cell filtration, while this has not happened to the mice immunized with pcDmzp3+MZP3 (see Fig. 11b).

High level of expression of IL-2, IL-12 and INF-γ are the characteristics of Th1 CD4+ T cells, and these immunoregulatory factors play important roles in several autoimmune diseases[21-26]. We examined whether there are changes to the expression of these cytokines in mice coimmunized with pcDmzp3+MZP3. Mice immunized with pcDNA3+MZP3 or pcDmzp3+OVA did not have their IL-12 and INF-γ expressions suppressed, while mice immunized with pcDmzp3+MZP3 had their IL-12 (see Fig. 11c) and INF-γ (see Fig. 11d) expressions suppressed. That implies that the mice coimmunized with pcDmzp3+MZP3 had working anti-inflammatory regulatory functions.

The T cell response is thought to have taken part in the occurrence of AOD, so we examined the T cells separated from mice spleens. These mice are first immunized with MZP3 protein and CFA, and immunized with pcDNA3, pcDNA3+MZP3, pcDmzp3, pcDmzp3+MZP3, pcDmzp3+OVA, and MZP3 14 days later. They are given enhanced immunization after another two weeks, and their spleens are taken out on the seventh day after the second immunization. These T cells are used to analyze the ability of responding to MZP3 protein antigen. T cells from the mice immunized with pcDmzp3+MZP3 basically have no amplification ability, while the T cells from other groups show very strong amplification ability (see 11e). These results show that AOD suppression after coimmunization with pcDmzp3+MZP3 is related to non-antigen-specific T cells response. Therefore, that means AOD suppression after coimmunization with pcDmzp3+MZP3 happened via reduction of the expression of inflammatory factors and suppression of T cells. In summary, AOD suppression is antigen-specific, as the mismatched combinations do not have the same results as those of coimmunization with pcDmzp3+MZP3.

MZP3 autoantibody plays an important role in guiding the distribution of autoimmune inflammation response, and can quickly worsen autoimmune diseases[13]. Therefore, our next task is to examine whether the coimmunization has inhibited production of anti-MZP3 antibody. The results show that the antibody titer is the same in all three groups, namely, the groups immunized with pcDmzp3+MZP3, pcDNA3+MZP3, and MZP3 (see Fig. 11f). The result is identical to that of previous studies. Without the T cell response, simply by transferring antibodies, autoimmune ovarian diseases cannot be induced[13].

2.6 Characteristics of regulatory T cells induced by qualitative coimmunization

Regulatory T cells can inhibit the T cell response and prevent the occurrence of autoimmune diseases. Cytokines IL-10 and FoxP3 play an important role in the process of T cells response suppression[21-29]. In order to examine whether coimmunization-induced regulatory T cells can express specific cytokines and signs, we have separated T cells from mice respectively immunized with pcDNA3, pcDNA3+MZP3, pcDmzp3, pcDmzp3+MZP3, pcDmzp3+OVA, and MZP3. These cytokines or signs get intracellular-stained with specific fluorescence-labeled antibodies, and analyzed by flow cytometry. T cells separated from mice immunized with pcDmzp3+MZP3 can express IL-10 and FoxP3 at a high level (see Fig. 12a). In contrast, the naive (control groups) can only express IL-10 and FoxP3 at a low level. Transcription factor FoxP3 is a specific factor of regulatory T cells[30,31], so we presume that the coimmunization may have induced regulatory T cells. We examine whether this type of regulatory T cell is CD4+CD25+ double positive. We have examined the expression status of CD25 and found that the expression of CD25 among different groups is basically the same (see Fig. 13).

Based on the aforementioned data, through coimmunization with DNA and protein of identical genes, we can possibly induce a type of CD4+CD25+ regulatory T cell, which is able to inhibit antigen-specific T cell response and prevent the occurrence of autoimmune diseases.

Discussion

Our studies have proven that coimmunization with MPZ3 DNA and protein induces a type of regulatory T cell, which is able to inhibit occurrence of autoimmune oophoritis. The phenotype of this regulatory T cell is CD4+CD25+Foxp3+, and it can express IL-10, inhibit antigen-specific T cell response and reduce the expression of cytokines IL-12 and INF-$\gamma$.

Zona pellucida proteins are very conservative in mammals. The AOD model established in mice can help better understand the pathology of autoimmune oophoritis in other animals, especially in humans. Through injection with 15 amino ZPP3 small peptide (328-342) and CFA on the sole and beneath the skin of B6AF1 mice [(C57BL/6 X 4/J) F1], autoimmune oophoritis is induced[12,13], while oophoritis has not happened to C57BL/6 mice. Through injection with MZP3 protein and CFA on the sole and muscle, we have managed to establish an oophoritis model in mice, although the disease is not as severe as previously reported[13]. Two reasons are assumed for the difference. (1) There are more T cell epitopes with the MZP3 protein that we used, and these epitopes have caused a stronger T cell immune response, and AOD is induced by the T cell response[12,]. (2) As we replace subcutaneous injection with muscular injection, more B cell epitopes have induced production of higher levels of autoimmune antibodies. Autoimmune antibodies can change the distribution of T cell-mediated inflammatory response and can worsen inflammatory response[13].

Natural regulatory T cells CD24+CD25+ can inhibit T cell response and maintain autoimmune tolerance. If the regulatory T cells are removed, diseases may occur[32,33,34], so these cells play rather important roles in preventing the occurrence of autoimmune diseases. These characteristics have made regulatory T cells a potential tool for treating autoimmune diseases[35]. Some of recent studies show that regulatory T cells are indeed able to treat autoimmune diseases, and that these regulatory T cells can be either non-antigen-specific[36,38] or antigen-specific[18,39]. We have also previously reported that coimmunization with pcD-VP1 and 146S antigen induced T cells response immunosuppression[19]. In the present study, we inhibit AOD in this way, that is, by coimmunization with MZP3 DNA and protein. Antigen-specific T cell response is inhibited, and expressions of cytokines IL-12 and INF-$\gamma$ are reduced, while the expression level of IL-10 and FoxP3 goes up. We infer that a type of antigen-specific regulatory T cell has been induced. Results of CD25 staining shows that this type of regulatory T cell induced might be CD25-. Therefore, we conclude that the coimmunization has induced antigen-specific immunosuppression, which is mediated by regulatory T cells CD4+CD25-FoxP3+IL10+.

In summary, we have proven a new method to treat autoimmune diseases. A new type of regulatory T cell may have been induced and it may have mediated immunosuppression of antigen-specific T cells. Therefore, further exploration of relevant mechanisms may help develop new methods to treat autoimmune diseases in human and animals.

References for the embodiments include:

1. S. L. Zou, C. Guo and X. P. Liu. (2000) The influence of Lake Beach's Environmental Evolution on the Yangtze Vole Disaster in Dongting Lake Area [J]. Journal of Natural Disasters, Vol. 9, No. 2, pp118-122.

2. W. K. Bao et al. Ecological Restoration and Rehabilitation: Development, Researching Features and Existing Major Problems [J]. World Sci-Tech R & D, Vol. 23, No. 1, pp 44-48.

3. Joseph Kim, Joo-Sung Yang, Kelledy H. etal. Modulation of antigen-specific 10 cellular immune responses to DNA vaccination in rhesus macaques through the use of IL-2, IFN-g, or IL-4 gene adjuvants [J]. Vaccine, 2001, 19 2496 - 2505.

4. Gwatkin, R. B. L. Effect of viruses on early mammalian development, I: action of Mengo Encephalitis Virus on mouse ova cultivated in vitro [J]. Proc. Natl. Acad. Sci. USA, 1963, 50: 576-581.

5. Baltz, J. M., Katz, R. A. The mechanics of sperm-egg interaction at the zona pellucida[J]. Biophys. J, 1988, 54 (4) :643-654.

6. Lloyd ML, Shellam GR, Papadimitriou JM, et al. Immunocontraception is induced in BALB/c mice inoculated with murine cytomegalovirus expressing mouse zona pellucida 3[J]. Biol Reprod, 2003, 68(6) :2024-2032

7. Dunbar, B. S., Bundman, D. S., Evidence for a role of the major glycoprotein in the structural maintenance of the pig zona pellucida[J]. J. Reprod. Fertil, 1987, 81, 363 - 376.

8. Burton K A, Vanee C C, Purcell K, Winship I, Shelling AN. Autosomal translocation associated with premature ovarian failure. J Med Genet 2000, 37: 25 1-2

9. Wheatcroft N, Weetman. Is premature ovarian failure an autoimmune disease? Autoimmunity 1997, 25:157 - 165

10. Hoek A, Schoemaker J, Drexhage HA. Premature ovarian failure and ovarian autoimmunity. Endocr Rev 1997, 18:107 - 134

11. Coulam CB, Kempers RD, Randall RV. Premature ovarian failure: evidence for the autoimmune mechanism. Fertil Steril 1981, 36:238 - 240

12. Rhim S H, Millar SE, Robey F, Luo A M, Lou Y H, Yule T, Allen P, Dean J, Tung K S K. Autoimmune disease of the ovary induced by a ZP3 peptide from the mouse zona pellucida. J Clin Invest 1992, 89:28 - 35

13. Lou Y, Park K, Agersborg S, Alard P, Tung K S K. Retargeting T Cell-Mediated

14. Sakaguchi, S. 2005. Naturally arising Foxp3-expressing CD25 (+) CD4 (+) regulatory T cells in immunological tolerance to self and nonself. Nat. Immunol. 6 : 345 - 352.

15. Viglietta, V., C. Baecher-Allan, H. L. Weiner, and D. A. Hafler. 2004. 5 Loss of functional suppression by CD4+CD25+ regulatory T cells in patients with multiple sclerosis. J. Exp. Med. 199:971 - 979.

16. Ehrenstein, M. R., J. G. Evans, A. Singh, S. Moore, G. Warnes, D. A. Isenberg, and C. Mauri. 2004. Compromised function of regulatory T cells in rheumatoid arthritis and reversal by anti-TNFalpha therapy. J. Exp. Med. 200 : 277 - 285.

17. Kriegel, M. A., T. Lohmann, C. Gabler, N. Blank, J. R. Kalden, and H. M. Lorenz. 2004. Defective suppressor function of human CD4+CD25+ regulatory T cells in autoimmune polyglandular syndrome type II. J. Exp. Med. 199:1285 - 1291.

18. Samy, E T, Parker L A, Sharp C P, Tung K S K. Continuous control of 15 autoimmune disease by antigen-dependent polyclonal CD4+ CD25+ regulatory T cells in the regional lymph node. JEM, 2005, 202: 771 - 781

19. Jin, H., Y. Kang, G. Zheng, Q. Xie, C. Xiao, X. Zhang, Y. Yu, K. Zhu, G. Zhao, F. Zhang, A. Chen, and B. Wang. 2005. Induction of active immune suppression by coimmunization with DNA- and protein-based vaccines. Virology 337 : 183-191.

20. Sambrook J, Fritsch EF, Maniatis T. Molecular Cloning: A Laboratory Manual, (2nd ed). New York: Cold Spring Harbor Laboratory Press, 1989. 19-56

21. Sharp C, Thompson C, Samy E T, Noelle R, Tung K S K. CD40 Ligand in Pathogenesis of Autoimmune Ovarian Disease of Day 3-Thymectomized Mice: Implication for CD40 Ligand Antibody Therapy. The Journal of Immunology, 2003, 170: 1667 - 1674.

22. Neurath, M. F., I. Fuss, B. L. Kelsall, E. Stuber, and W. Strober. 1995. Antibodies to interleukin 12 abrogate established experimental colitis in mice. J. Exp. Med. 182:1281.

23. Leonard, J. P., K. E. Waldburger, and S. J. Goldman. 1995. Prevention of experimental autoimmune encephalomyelitis by antibodies against interleukin 12. J. Exp. Med. 181:381.

24. Tarrant, T. K., P. B. Silver, J. L. Wahlsten, L. V. Rizzo, C. C. Chan, B. Wiggert, and R. R. Caspi. 1999. Interleukin 12 protects from a T helper type 1-mediated autoimmune disease, experimental autoimmune uveitis, through a mechanism involving interferon $\gamma$, nitric oxide, and apoptosis. J. Exp. Med. 189:219

25. McIntyre, K. W., D. J. Shuster, K. M. Gillooly, R. R. Warrier, S. E. Connaughton, L. B. Hall, L. H. Arp, M. K. Gately, and J. Magram. 1996. Reduced incidence and severity of collagen-induced arthritis in interleukin-12-deficient mice. Eur. J. Immunol. 26:2933.

26. Okura, Y., K. Takeda, S. Honda, H. Hanawa, H. Watanabe, M. Kodama, T. Izumi, Y. Aizawa, S. Seki, and T. Abo. 1998. Recombinant murine interleukin-12 facilitates induction of cardiac myosin-specific type 1 helper T cells in rats. Circ. Res 82:1035.

27. Fontenot, J.D., M.A. Gavin, and A. Y. Rudensky. 2003. Foxp3 programs the development and function of CD4+CD25+ regulatory T cells. NatImuncd4:330-336.

28. Roncarolo, M.G., R. Bacchetta, C. Bordignon, S. Narula, and M. K. Levings. 2001. Type 1 T regulatory cells. Immunol Rev 182:68-79.

29. Stock, P., 0. Akbari, G. Berry, G. J. Freeman, R. H. Dekruyff, and D. T. Umetsu. 2004. Induction of T helper type 1-like regulatory cells that express 15 Foxp3 and protect against airway hyper-reactivity. Nat Immunol 5:1149-1156.

30. Khattri, R., D. Kasprowicz, T. Cox, M. Mortrud, M. W. Appleby, M. E. Brunkow, S.F. Ziegler, and F. Ramsdell. 2001. The Amount of Scurfin Protein Determines Peripheral T Cell Number and Responsiveness. JImmunol 167:6312-6320.

31. Khattri, R., T. Cox, S. A. Yasayko, and F. Ramsdell. 2003. An essential role for Scurfin in CD4+CD25+ T regulatory cells. Nat IMMI.11101 4:337-342.

32. Sakaguchi S, Sakaguchi N, Asano M, Itoh M, Toda M. Immunologic self tolerance maintained by activated T cells expressing IL-2 receptor achains. J Immunol 1995; 155: 1151-64.

33. Sakaguchi S, Takahashi T, Nishizuka Y. Study on cellular events in 25 postthymectomy autoimmune oophoritis in mice. I. Requirement of Lyt-1 effector cells for oocytes damage after adoptive transfer. J Exp Med 1982; 156: 1565-76.

34. Asano M, Toda M, Sakaguchi N, Sakaguchi S. Autoimmune disease as a consequence of developmental abnormality of a T cell subpopulation. J Exp Med 1996; 184: 387-96.

35. Nielsen J, Holm T L and Claesson M H. CD4+CD25+ regulatory T cells: II. Origin, disease models and clinical aspects. APHIS 112: 642-50, 2004

36. KohmAP, Carpentier PA, Anger HA, Miller SD. Cutting edge: CD4+CD25+ regulatory T cells suppress antigen-specific autoreactive immune responses and central nervous system inflammation during active experimental autoimmune encephalomyelitis. J Immunol. 2002;169:4712-4716.

37. Zhang X, Koldzic DN, Izikson L, et al. IL-10 is involved in the suppression of experimental autoimmune encephalomyelitis by CD25(+)CD4(+) regulatory T cells. Int Immunol. 2004;16:249-256.

38. Mottet C, Uhlig HH, Powrie F. Cutting edge: cure of colitis by CD4+CD25+ regulatory T cells. J Immunol. 2003;170:3939-3943.

39. Tang Q, Henriksen KJ, Bi M, et al. In vitro-expanded antigen-specific regulatory T cells suppress autoimmune diabetes. J Exp Med. 2004; 199:1455-1465.

Embodiment 4: treating/preventing multiple sclerosis by coimmunization (not part of the invention)

Multiple sclerosis (MS) is a typical central nervous system inflammatory demyelinating disease, and an autoimmune disease. The most common animal model for studies of MS is called experimental allergic encephalomyelitis (EAE). Animals' EAE clinical manifestation and pathological changes are extremely similar to that of acute multiple sclerosis. Therefore, a successfully established EAR animal model is the precondition to study EAE.

The objective of this experiment is to treat or prevent EAE in EAE mice model based on coimmunization-induced immunosuppression, and to further explore immune mechanisms related to MS outset during the process of the study.

The protein autoantigens of multiple sclerosis (MS) include myelin oligodendrocyte glycoprotein (MOG) and two myelin antigens (myelin basic protein (MBP) and PLP (protein lipoprotein)).

Existing experiment results are limited to inducement of EAE mice model and construction of related MOG genetic DNA without more in-depth exploration. The preliminary results are as follows.

1. Cloning and expression of MOG35-55 2copies genes

1.1 Materials and reagents

pMD18-T sequencing vector is purchased from the Takara Company, and Escherichia coli DH5αbacterial strain is the conserved strain from our laboratory. PCR product recycling kit, DNA marker, restriction enzyme, exTaq enzyme, and RT-PCT and PCR primers are all purchased from the Takara Company. The sequencing kit is purchased from PE (USA) Company, and the other reagents are analytical reagents.

1.2 Method

1.2.1 Cloning of MOG35-55 genes

The commonly used autoantigens for inducement of animal EAE model are 35-55 amino acids of myelin oligodendrocyte glycoprotein (MOG). The amino sequence is:

MEVGWYRSPFSRVVHLYRNGK, and the DNA sequence is GAGGTGGGTTGGTACCGTTCCCTTCTCAAGAGTGGTTCACCTCTACCGAAATGGCAA G.

According to the gene sequence, design synthetic primers and DNA fragments, and construct plasmid DNA of two copies of MOG35-55 by overlapping PCR.

Design of overlapped DNA fragments:

Fragment 1: 5' - ATGGAGGTGGGTTGGTACCGTTCTCCCTTC TCAAGAGTGG-3'

Fragment 2: 5' - CCTCCATCTTGCCATTTCGGTAGAGGTGAACCACTCTTGAGAAGGGAGAA-3'

Fragment 3:5'-CCGAAATGGCAAGATGGAGGTGGGTTGGTACCGTTCTCCCTTCTCAAGAG-3'

Fragment 4: 5' -TTACTTGCCA TTTCGGTAGA GGTGAACCAC TCTTGAGAAG GGAGAACGG-3 '

Design of primers:

MOG35 P1: 5' - AAGGATCCATGGAGGTGGGTTG -3'

MOG35 P2: 5' - GCTCTAGATTACTTGCCATTTC -3'

DNA and primers designed for PCR reaction, amplification parameters: 94°C 2 min; 94°C 30s, 60°C 40s, and 72°C 35s. 35 cycles; 72°C 10 min, take reaction products for agarose gel electrophoresis analysis. Follow the instructions of Takara's PCR Fragment Recovery Kit to recycle PCR products. At 16°C and under the effect of $T_4$ DNA ligase, leave 2 copies of the recycled MOG35-55 fragment and pMD 18-T vector overnight and connect them. The constructed plasmid is named T-MOG352c. The connection product transfers competent cells of the DH5α bacterial strain. Extract a small quantity of plasmid and use EcoR I and Hind III to do double enzyme digestion of recombinant plasmid. After the enzyme digestion reaction, do agarose gel electrophoresis analysis. In order to identify the sequence of cloned DNA, purify the recombinant plasmid and do further sequencing. Then use DNAMAN software to analyze the results.

1.2.1 Construction of eukaryon expression vector and transient expression of recombinant in BHK21 cells

Do BamH and Hind III double enzyme digestion of plasmids that are correct according to sequencing, and connect them to the corresponding enzyme digested eukaryon expression vector pVAX. The vector is named pVAXMOG352c. Do the eukaryon transfection experiment with recombinant plasmids that identified by sequencing as correct. Refer to lipofectamine instruction for specific procedures of transfection. By lipofection, use pVAXMOG352c to transfer BHK21 cells. Collect the cells after 48 hours from transfection, extract the complete RNA, and use MOG35 P1 and MOG35 P2 as primers to examine the expression of the target genes by RT-PCR.

1.3 Results of experiment

The results of enzyme digestion of plasmid T-MOG352c are shown in Fig. 14. The results of enzyme digestion of plasmid pVAXMOG352c are shown in Fig. 15. The results of transient expression of plasmid pVAXMOG352c in BHK21 cells are shown in Fig. 16.

2. Inducement and identification of EAE animal model

2.1 Procedures of direct immunization

Take C57BL/6 mice, underneath the skin of their back, immunize them with 200μg of fully CFA-emulsified small peptide MOG35-55 (1μg/μl, 200μl, and containing 750μg of tubercle bacillus BCG), at the end day of the immunization and at the end of the second day of the immunization, give the mice tubercle bacillus BCG of Bordetella pertussis (respectively $10^8$- and $10^9$), and give an enhanced immunization with 200μg of MOG 7 days later.
Scoring criteria:

0 - No clinical symptoms are shown.
1 - The animal's tail becomes powerless, and feeble.
2 - The animal's tail becomes powerless, and it becomes feeble at its forelimbs or hind limbs.
3 - The animal becomes seriously feeble at its forelimbs or hind limbs, and cannot return to its original posture after being manually turned over.
4 - The animal's limbs become numb and it cannot return to its original posture after being manually turned over.
5 - The animal reaches moribund conditions.

The results are shown in Fig. 17 with different colors representing different individual mice: Severe incidence after 40 days (incidence index > 2) is up to 70%.

□ For the most recently immunized batch, incidence (incidence index at 4) after five weeks from the initial immunization is 5 out of 20 mice.

2.2 Model of adoptive-transferred T cells from morbid mice

Fig. 18 shows negative mice get subcutaneous injection immunization of 200μg of CFA-emulsified MOG antigen, after getting adoptive-transferred T cells from morbid mice spleen;
Fig. 19 shows negative mice get subcutaneous injection immunization of 200μg of CFA-emulsified MOG antigen, after getting adoptive-transferred T cells from morbid mice lymph node.

Conclusion: Mice getting adoptive-transferred T cells from the lymph node have earlier disease onset than the mice getting such T cells from the spleen, with more steady symptomatic changes. Mice getting adoptive-transferred T cells from the spleen have a higher incidence index.

2.3 Examination of some physical signs with morbid mice

Fig. 20 shows the T cell amplification status to be specific to the MOG autoantigen after the disease onset.
Conclusion: The peripheral immune organ of morbid mice have strong T cell amplification reaction specific to MOG autoantigens.
Fig. 21 shows expression status of some cytokines inside the T cells specific to MOG autoantigens after the mice are induced to have disease onset.

3. Treating EAE mice by coimmunization

3.1 Strategies

Following procedure 2.1, use C57 mice the inducement EAR model, and keep track of clinical symptoms until all of the mice have a clinical index above 3. Group the morbid mice as follows and immunize them separately by intramuscular injection.

1. Immunize with 100μl of MOG35-55 peptide (fully emulsified with CFA at 1:1 until the concentration is 1μg/μl)
2. Immunize with 100μl of pVAXMOG352c plasmid DNA (concentration at 1μg/μl)
3. Immunize with 100μl each of pVAXMOG352cMOG35-55 peptide and pVAXMOG352c (same concentration as above)
4. Immunize with 100μl each of pVAXMOG352cMOG35-55 peptide and pVAX1 (same concentration as above)
5. No-treatment group

Enhanced immunization is given 14 days later.
Since the initial immunization, observe and record clinical symptoms and score them. After 7 days from the enhanced immunization, take spleens from each group of mice under aseptic condition, and do the T cell amplification experiment by MTT. At the same time, take the mice's cerebrum, cerebellum, and spinal cord, fix the tissues with 4% paraformaldehyde, and proceed with preparation of tissue section and microscopic examination of pathological changes.
Examination of cytokines including IL-1, IL-2, INF-γ, IL-4, IL-10, FoxP3, IL17, and TGF, etc.: Group the expressions into expressions of spleen cytokines and expressions of cytokines in cerebrospinal fluid.
The next experiment is to verify the application of coimmunization by immunological experiment based on well-developed animal model.

序列表

<160> 1

<210> 1
<211> 15
<212> PRT
<213> synthetic

<220>
<223>

<400> 1

Ser His Leu Val Glu Ala Leu Tyr Leu Val Cys Gly Glu Arg Gly
　1　　　　　　　　　5　　　　　　　　　10　　　　　　　　　15

**Claims**

1. A vaccine for use in preventing and/or treating type 1 diabetes mellitus in a mammal, the vaccine comprising a mixture of:

   (a) a protein selected from the group consisting of an autoantigen causing the autoimmune disease and an immunogenic epitope polypeptide thereof with SEQ ID NO:1; and
   (b) a recombinant eukaryotic expression vector comprising an insert that encodes the autoantigen or the immunogenic epitope polypeptide of the autoantigen with SEQ ID NO:1,

   wherein the autoantigen is insulin;
   wherein the vaccine stimulates regulatory T cells to inhibit an antigen-specific T cell response.

2. A vaccine for use in preventing and/or treating autoimmune oophoritis in a mammal, the vaccine comprising a mixture of:

   (a) a protein consisting of an autoantigen causing the autoimmune disease; and
   (b) a recombinant eukaryotic expression vector comprising an insert that encodes the autoantigen,

   wherein the autoantigen is zona pellucida 3;
   wherein the vaccine stimulates regulatory T cells to inhibit an antigen-specific T cell response.

3. The vaccine for use of claim 1, wherein:

   (a) the protein is the autoantigen and the insert encodes the autoantigen;
   (b) the protein is the autoantigen epitope polypeptide of SEQ ID NO:1 and the insert encodes the protein autoantigen epitope polypeptide of SEQ ID NO:1
   (c) the protein is the autoantigen and the insert encodes the autoantigen epitope polypeptide of SEQ ID NO:1 or
   (d) the protein is the autoantigen epitope polypeptide of SEQ ID NO:1 and the insert vector encodes the autoantigen; and

   wherein the autoantigen is insulin.

4. The vaccine for use according to claim 1 wherein the autoantigen comes from a mammal selected from the group consisting of a human, a dog, and a cat.

5. The vaccine for use of claim 4 wherein the autoantigen is human insulin.

6. The vaccine for use of claim 3 wherein the vector is a mammalian expression vector.

7. The vaccine for use of claim 6 wherein the vector is selected from the group consisting of pcDNA3.0, pVAX1, and provax.

8. The vaccine for use of claim 7 wherein the protein is human insulin, wherein the insert encodes insulin, and wherein the vector is pVAX.

9. The vaccine for use of claim 7 wherein the protein is human insulin fragment B9-23 with SEQ ID No:1, wherein the insert encodes human insulin fragment B9-23 with SEQ ID NO:1, and wherein the vector is pcDNA3.0.

10. The vaccine for use of claim 3, wherein the mass ratio of the protein to the vector is 5:1 to 1:5.

11. The vaccine for use of claim 3, wherein the mass ratio of the protein to the vector is 1:1 to 1:2.


**Patentansprüche**

1. Impfstoff zur Verwendung bei der Prävention und/oder Behandlung von Diabetes mellitus Typ 1 bei einem Säuger, wobei der Impfstoff ein Gemisch aus

(a) einem Protein, ausgewählt aus der Gruppe bestehend aus einem Autoantigen, das die Autoimmunkrankheit verursacht, und einem Polypeptid davon, das ein immunogenes Epitop darstellt, mit SEQ ID NO:1; und
(b) einem rekombinanten eukaryotischen Expressionsvektor, der ein Insert umfasst, welches das Autoantigen oder das Polypeptid des Autoantigens, das ein immunogenes Epitop darstellt, mit SEQ NO:1 codiert, umfasst,

wobei es sich bei dem Autoantigen um Insulin handelt;
wobei der Impfstoff regulatorische T-Zellen stimuliert, um eine antigenspezifische T-Zell-Antwort zu hemmen.

2. Impfstoff zur Verwendung bei der Prävention und/oder Behandlung von Autoimmun-Oophoritis bei einem Säuger, wobei der Impfstoff ein Gemisch aus

(a) einem Protein, bestehend aus einem Autoantigen, das die Autoimmunkrankheit verursacht, und
(b) einem rekombinanten eukaryotischen Expressionsvektor, der ein Insert umfasst, welches das Autoantigen codiert, umfasst,

wobei es sich bei dem Autoantigen um Zona pellucida 3 handelt;
wobei der Impfstoff regulatorische T-Zellen stimuliert, um eine antigenspezifische T-Zell-Antwort zu hemmen.

3. Impfstoff zur Verwendung nach Anspruch 1, wobei:

(a) es sich bei dem Protein um das Autoantigen handelt und das Insert das Autoantigen codiert;
(b) es sich bei dem Protein um das Autoantigen-Epitop-Polypeptid der SEQ NO:1 handelt und das Insert das Protein-Autoantigen-Epitop-Polypeptid der SEQ ID NO:1 codiert,
(c) es sich bei dem Protein um das Autoantigen handelt und das Insert das Autoantigen-Epitop-Polypeptid der SEQ ID NO:1 codiert, oder
(d) es sich bei dem Protein um das Autoantigen-Epitop-Polypeptid der SEQ NO:1 handelt, und der Insert-Vektor das Autoantigen codiert; und

wobei es sich bei dem Autoantigen um Insulin handelt.

4. Impfstoff zur Verwendung nach Anspruch 1, wobei das Autoantigen aus einem Säuger stammt, der aus der Gruppe ausgewählt ist, die aus einem Menschen, einem Hund und einer Katze besteht.

5. Impfstoff zur Verwendung nach Anspruch 4, wobei es sich bei dem Autoantigen um Humaninsulin handelt.

6. Impfstoff zur Verwendung nach Anspruch 3, wobei es sich bei dem Vektor um einen Säuger-Expressionsvektor handelt.

7. Impfstoff zur Verwendung nach Anspruch 6, wobei der Vektor aus der Gruppe ausgewählt ist, die aus pcDNA3.0, pVAX1, und Provax ausgewählt ist.

8. Impfstoff zur Verwendung nach Anspruch 7, wobei es sich bei dem Protein um Humaninsulin handelt, wobei das Insert Insulin codiert, und wobei es sich bei dem Vektor um pVAX handelt.

9. Impfstoff zur Verwendung nach Anspruch 7, wobei es sich bei dem Protein um Humaninsulinfragment B9-23 mit SEQ ID NO:1 handelt, wobei das Insert Humaninsulinfragment B9-23 mit SEQ ID NO:1 codiert, und wobei es sich bei dem Vektor um pcDNA3.0 handelt.

10. Impfstoff zur Verwendung nach Anspruch 3, wobei das Massenverhältnis zwischen dem Protein und dem Vektor 5:1 bis 1:5 beträgt.

11. Impfstoff zur Verwendung nach Anspruch 3, wobei das Massenverhältnis zwischen dem Protein und dem Vektor 1:1 bis 1:2 beträgt.

**Revendications**

1. Vaccin pour utilisation dans la prévention et/ou le traitement du diabète sucré de type 1 chez un mammifère, le vaccin comprenant un mélange de :

(a) une protéine choisie dans le groupe constitué d'un auto-antigène causant la maladie auto-immune et un polypeptide d'épitope immunogène de celui-ci avec SEQ ID NO: 1 ; et
(b) un vecteur d'expression eucaryote recombinant comprenant un insert qui code pour l'auto-antigène ou le polypeptide d'épitope immunogène de l'auto-antigène avec SEQ ID NO: 1,

dans lequel l'auto-antigène est l'insuline ;
le vaccin stimulant des lymphocytes T régulateurs pour inhiber une réponse de lymphocytes T spécifiques à un antigène.

2. Vaccin pour utilisation dans la prévention et/ou le traitement d'une ovarite auto-immune chez un mammifère, le vaccin comprenant un mélange de :

(a) une protéine constituée d'un auto-antigène causant la maladie auto-immune ; et
(b) un vecteur d'expression eucaryote recombinant comprenant un insert qui code pour l'auto-antigène,

dans lequel l'auto-antigène est zona pellucida 3 ;
le vaccin stimulant des lymphocytes T régulateurs pour inhiber une réponse de lymphocytes T spécifiques à un antigène.

3. Vaccin pour utilisation de la revendication 1, dans lequel :

(a) la protéine est l'auto-antigène et l'insert code pour l'auto-antigène ;
(b) la protéine est le polypeptide d'épitope d'auto-antigène de SEQ ID NO: 1 et l'insert code pour le polypeptide d'épitope d'auto-antigène protéique de SEQ ID NO: 1,
(c) la protéine est l'auto-antigène et l'insert code pour le polypeptide d'épitope d'auto-antigène de SEQ ID NO: 1, ou
(d) la protéine est le polypeptide d'épitope d'auto-antigène de SEQ ID NO: 1 et le vecteur d'insert code pour l'auto-antigène ; et

dans lequel l'auto-antigène est l'insuline.

4. Vaccin pour utilisation selon la revendication 1 dans lequel l'auto-antigène provient d'un mammifère choisi dans le groupe constitué d'un humain, un chien et un chat.

5. Vaccin pour utilisation de la revendication 4 dans lequel l'auto-antigène est l'insuline humaine.

6. Vaccin pour utilisation de la revendication 3 dans lequel le vecteur est un vecteur d'expression de mammifère.

7. Vaccin pour utilisation de la revendication 6 dans lequel le vecteur est choisi dans le groupe constitué de pcDNA3.0, pVAX1 et provax.

8. Vaccin pour utilisation de la revendication 7 dans lequel la protéine est l'insuline humaine, dans lequel l'insert code pour l'insuline et dans lequel le vecteur est pVAX.

9. Vaccin pour utilisation de la revendication 7 dans lequel la protéine est le fragment d'insuline humaine B9-23 avec SEQ ID NO: 1, dans lequel l'insert code pour le fragment d'insuline humaine B9-23 avec SEQ ID NO: 1 et dans lequel le vecteur est pcDNA3.0.

10. Vaccin pour utilisation de la revendication 3, dans lequel le rapport en masse de la protéine au vecteur est de 5:1 à 1:5.

11. Vaccin pour utilisation de la revendication 3, dans lequel le rapport en masse de la protéine au vecteur est de 1:1 à 1:2.

Fig 1a

Fig. 1b

Fig 1c

8.94%

M1

B9-23 immunization group

22.32%

M1

pcDB9-23 immunization group

0.08%

M1

pcDB9-23 & 9-23 coimmunization group

Fig 2a

97.36%

B9-23 immunization group

96.48%

M1

pcDB9-23 immunization group

23.83%

M1

pcDB9-23 & B9-23 coimmunization group

Fig 2b

**Naïve**

**pVAX-insulin** and human insulin protein coimmunization group

**human insulin protein immunization group**

**pVAX1** immunization group

**pVAX-insulin & VP1** coimmunization group

**pVAX-insulin** immunization group

**pVAX1** and human insulin protein coimmunization group

Fig 3a

Fig 3b

Fig. 3c

Number of Weeks after 2nd Immunization (Week)

Fig. 4

Naive Mice

Morbid DM Mice

pVAX-Insulin and human insulin protein coimmunization group

Fig. 5

Fig. 6a

MZP3      DALVYSTFILEDPRFVSGLSIIRTNRVEVPIECRYPFQGN   146
           DALVYSTFILHDPRFVGNLSIVRTNRASIPIECRYPRRGN   147
Marmosets  DALVYSTFILHDPRFVGNLSIVRTNRASIPIECRYPRQGX   200
Human      NALVYSTFLIHSPRFAGNLSIIRTNRADVPIECRYPFHSN   145
           DALVYSTFLLHNPRFMGNLSIILRTIRABVPIECRYPPHSN   145
Canis      DALVYSTFIRHDPRFAGNLSILRTNRABVPIECHYPRQGN   146
familiaris   NALVYSTFILHNPRFAGNLSILRTNRADVPIECHYPRQGN   146
           alvystfl  h  prp    lsi  rtnr  e  piec  ypr    n

MZP3      VSSHFIQFTVVFFFAIVSSEEKLASSLRLMEBNUNTERSA   186
           VSSQAILPTWIPFRTIVFSEEKITESLRLMEBNUSTERRT   187
Marmosets  VSSQAILPTWIPFRTIVFSEEKITESLRLMEBNUNAERRS   240
Human      VSSQAILPTWVPFRTIMLFCEEKIVFSLRLMEBDUGSEKQS   185
           VSSEAILPTWVPFTTMLSEEKIVFSLRLMEBDUGSEKQS   185
Canis      VSSWAILPTWVPFSTIVFSEEKIVFSLRLMEBNUSAEKMT   186
familiaris   VSSWAIQPTRVPFTTIVFSEEKIVFSLRLMEBNQSAEKMT   186
           vss    i ptw pfr t    eekl fslrlmee w  ek

MZP3      PTFHLGEVAHLQAEVQIGSHLPLQLFVDHCVASPSPIPEP   226
           PTFHLGDVAHLQAEIHIGSIVPLRLFVDHCVATHI..PIQ   225
Marmosets  PTFHLGDRAHLQAEIHIGSIVPLRLFVDHCVATHI..PIQ   278
Human      PTFQLGDIAHLQAEVHIGSHMFLRLFVDHCVPTLI..PDR   223
           PTFQLGDIAHLQAEVHIGRHIPLRLFVDICVATLI..PLQ   223
Canis      PTFQLGDRAHLQAEVHIGSIVPLRLFVDHCVATLI..PDW   224
familiaris   PTFQLGDRAHLQAEVHIGSHVPLRLFVDHCVASLI..PLW   224
           ptf lg  ahlqa     tg h pl lfvd cva       pd

MZP3      NSSIYFTIVDFHGCLVDGISESFSAHQVPRLRGEILQFTV   266
Marmosets  NASEYFTIVDFHGCLVDGITDASSAFQAPRLRGDILQFTV   265
           NASEYFTIVDFHGCLVDGITDASSAFKVPRLRGDILQFTV   318
Human      NAFHHEKIVDFHGCLVDGLYNSSSAFKAPRERGEILQFTV   263
           NASEEFTIVDFHGCLVDGLSDASSAFKAPRERGDILQFTV   263
Canis      NTSFSFTIVDFHGCLVDGITEASSAFKAPRLRGEILQFTV   264
familiaris   STSIYFTIVDFHGCLVDGITDASSAFKAPRERGEILQFTV   264
           p h' ivdfhgclvdgl     saf  prp p  lqftv

MZP3      DVFHFANSGRNTIYITCHLKVAPANQIPDKLNKACSFNNT   306
           DVFHFNDSRNMIYITCHLKVTLADQDPDELNKACSFSNA   305
Marmosets  DVFHFNDSRNMIYITCHLKVELADQDPDELNKACSFSNP   358
Human      DVFHFKDSRNTIYITCHLKVTPADRVPDQLNKACSFIRS   303
           YTFHFANDRRNMIYITCHLKVTPASRVPDQLNKACSFIRS   303
Canis      DVFHFANDSRNTIYITCHLKVTPADRVPDQLNKACSFSHS   304
familiaris   DVFHFANDISRNMIYITCHLKVTEVDRVPIQLNKACSFSHS   304
           f fa     rn  yitchlkv      pd lnkacsf k

Fig. 6b

a     1     2     M       M     1     2     b

Fig. 7

Fig. 8

Fig 9a

Fig. 9b

100x          400x

Naïve

CFA

MZP3

Fig. 9c

naïve          CFA          MZP3

0.23%          0.74%          11.15%

M1          M1          M1

FL1-H          FL1-H          FL1-H

CFSE

Fig. 9d

Naïve CFA  MZP3

IL-2

HPRT

Fig. 10a

Fig.10b

Fig.10c

Fig.11a

Fig.11b

Fig. 11c

Fig. 11d

Fig.11e

Fig.11f

Fig.12a

Fig. 12b

Fig. 14

Fig.15

Fig.16

Fig.17

Fig.18

Fig. 19

Fig. 20

Fig. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1716863 A


**Non-patent literature cited in the description**

- Report of the Expert Committee on the Diagnosis and Classification of Diabetes Mellitus. **ADA.** Diabetes Mellitus Care. American Diabetes Mellitus Association, 1997, vol. 20, 1183-1197
- **ATKINSON MA ; LEITER EH.** The NOD Mouse Model of Type I Diabetes Mellitus: As good as it gets?. *Nature,* 1999, vol. 5, 601-604
- **ATKINSON MA ; MACLAREN NK.** The pathogenesis of insulin-dependent diabetes mellitus. *N Engl J Med,* 1994, vol. 331, 1428-1436
- **BENOIST C ; MATHIS D.** Autoimmune diabetes mellitus: Retrovirus as trigger, precipitator or marker?. *Nature,* 1997, vol. 388, 833-834
- **BJORK S.** The cost of diabetes mellitus and diabetes mellitus care. *Diabetes mellitus Res Clin Pract,* 2001, vol. 54 (13-18
- **ITAMAR RAZL ; ROY ELDOR2 ; YAAKOV NAPARSTEK.** Immune Modulation for Prevention of Type I diabetes Mellitus. *TRENDS in Biotechnology,* 2005, vol. 23, 128
- **LONG XIURONG ; DU WENBIN ; SU ZHONGPU ; WEI QINGZHENG.** GAD-Ab Inspection in Children with Diabetes Mellitus. *Chinese Journal of Pediatrics,* 1998, vol. 10
- **EVERY et al.** *J Immunol.,* 2006, vol. 176, 4608-4615
- **JUEDES ; HERRATH.** *Diabetes Metab Res Rev,* 2004, vol. 20, 446-451
- **TU YIXIAN ; JIN HUALI ; ZHANG XINYU ; YANG RUOYE ; YANG FU ; ZHANG FUCHUN ; WANG BIN.** Comparisons of the Expression Efficiencies and Immune Effects of Two Eukaryotic Vectors Encoding CFSV E2 Gene. *Journal of China Agricultural University,* 2005, vol. 10 (6), 37-41
- **SAMBROOK J ; FRITSCH EF ; MANIATIS T.** Molecular Cloning: A Laboratory Manual. Cold Spring Harbor Laboratory Press, 1989, 19-56
- **JIN HUALI ; ZHANG FUCHUN ; SHAN WENJUAN ; ZHANG AILIAN ; LI YIJIE ; WANG BIN.** Expression of FMDV VP1 Protein in Pichia Pastoris and Its Immunological Activity in Mice. *Cellular and Molecular Immunology,* 2004, vol. 20 (5), 513-516

- **S. L. ZOU ; C. GUO ; X. P. LIU.** The influence of Lake Beach's Environmental Evolution on the Yangtze Vole Disaster in Dongting Lake Area [J. *Journal of Natural Disasters,* 2000, vol. 9 (2), 118-122
- **W. K. BAO et al.** Ecological Restoration and Rehabilitation: Development, Researching Features and Existing Major Problems [J. *World Sci-Tech R & D,* vol. 23 (1), 44-48
- **JOSEPH KIM ; JOO-SUNG YANG ; KELLEDY H et al.** Modulation of antigen-specific 10 cellular immune responses to DNA vaccination in rhesus macaques through the use of IL-2, IFN-g, or IL-4 gene adjuvants [J. *Vaccine,* 2001, vol. 19, 2496-2505
- **GWATKIN, R. B. L.** Effect of viruses on early mammalian development, I: action of Mengo Encephalitis Virus on mouse ova cultivated in vitro [J. *Proc. Natl. Acad. Sci. USA,* 1963, vol. 50, 576-581
- **BALTZ, J. M. ; KATZ, R. A.** The mechanics of sperm-egg interaction at the zona pellucida[J. *Biophys. J,* 1988, vol. 54 (4), 643-654
- **LLOYD ML ; SHELLAM GR ; PAPADIMITRIOU JM et al.** Immunocontraception is induced in BALB/c mice inoculated with murine cytomegalovirus expressing mouse zona pellucida 3[J. *Biol Reprod,* 2003, vol. 68 (6), 2024-2032
- **DUNBAR, B. S. ; BUNDMAN, D. S.** Evidence for a role of the major glycoprotein in the structural maintenance of the pig zona pellucida[J. *J. Reprod. Fertil,* 1987, vol. 81, 363-376
- **BURTON K A ; VANEE C C ; PURCELL K ; WINSHIP I ; SHELLING AN.** Autosomal translocation associated with premature ovarian failure. *J Med Genet,* 2000, vol. 37 (25), 1-2
- **WHEATCROFT N ; WEETMAN.** Is premature ovarian failure an autoimmune disease?. *Autoimmunity,* 1997, vol. 25, 157-165
- **HOEK A ; SCHOEMAKER J ; DREXHAGE HA.** Premature ovarian failure and ovarian autoimmunity. *Endocr Rev,* 1997, vol. 18, 107-134
- **COULAM CB ; KEMPERS RD ; RANDALL RV.** Premature ovarian failure: evidence for the autoimmune mechanism. *Fertil Steril,* 1981, vol. 36, 238-240

- **RHIM S H ; MILLAR SE ; ROBEY F ; LUO A M ; LOU Y H ; YULE T ; ALLEN P ; DEAN J ; TUNG K S K.** Autoimmune disease of the ovary induced by a ZP3 peptide from the mouse zona pellucida. *J Clin Invest,* 1992, vol. 89, 28-35
- **LOU Y ; PARK K ; AGERSBORG S ; ALARD P ; TUNG K S K.** *Retargeting T Cell-Mediated*
- **SAKAGUCHI, S.** Naturally arising Foxp3-expressing CD25 (+) CD4 (+) regulatory T cells in immunological tolerance to self and nonself. *Nat. Immunol.,* 2005, vol. 6, 345-352
- **VIGLIETTA, V. ; C. BAECHER-ALLAN ; H. L. WEINER ; D. A. HAFLER.** 5 Loss of functional suppression by CD4+CD25+ regulatory T cells in patients with multiple sclerosis. *J. Exp. Med.,* 2004, vol. 199, 971-979
- **EHRENSTEIN, M. R. ; J. G. EVANS ; A. SINGH ; S. MOORE ; G. WARNES ; D. A. ISENBERG ; C. MAURI.** Compromised function of regulatory T cells in rheumatoid arthritis and reversal by anti-TNFalpha therapy. *J. Exp. Med.,* 2004, vol. 200, 277-285
- **KRIEGEL, M. A. ; T. LOHMANN ; C. GABLER ; N. BLANK ; J. R. KALDEN ; H. M. LORENZ.** Defective suppressor function of human CD4+CD25+ regulatory T cells in autoimmune polyglandular syndrome type II. *J. Exp. Med.,* 2004, vol. 199, 1285-1291
- **SAMY, E T ; PARKER L A ; SHARP C P ; TUNG K S K.** Continuous control of 15 autoimmune disease by antigen-dependent polyclonal CD4+ CD25+ regulatory T cells in the regional lymph node. *JEM,* 2005, vol. 202, 771-781
- **JIN, H. ; Y. KANG ; G. ZHENG ; Q. XIE ; C. XIAO ; X. ZHANG ; Y. YU ; K. ZHU ; G. ZHAO ; F. ZHANG.** Induction of active immune suppression by coimmunization with DNA- and protein-based vaccines. *Virology,* 2005, vol. 337, 183-191
- **SHARP C ; THOMPSON C ; SAMY E T ; NOELLE R ; TUNG K S K.** CD40 Ligand in Pathogenesis of Autoimmune Ovarian Disease of Day 3-Thymectomized Mice: Implication for CD40 Ligand Antibody Therapy. *The Journal of Immunology,* 2003, vol. 170, 1667-1674
- **NEURATH, M. F. ; I. FUSS ; B. L. KELSALL ; E. STUBER ; W. STROBER.** Antibodies to interleukin 12 abrogate established experimental colitis in mice. *J. Exp. Med.,* 1995, vol. 182, 1281
- **LEONARD, J. P. ; K. E. WALDBURGER ; S. J. GOLDMAN.** Prevention of experimental autoimmune encephalomyelitis by antibodies against interleukin 12. *J. Exp. Med.,* 1995, vol. 181, 381
- **TARRANT, T. K. ; P. B. SILVER ; J. L. WAHLSTEN ; L. V. RIZZO ; C. C. CHAN ; B. WIGGERT ; R. R. CASPI.** Interleukin 12 protects from a T helper type 1-mediated autoimmune disease, experimental autoimmune uveitis, through a mechanism involving interferon $\gamma$, nitric oxide, and apoptosis. *J. Exp. Med.,* 1999, vol. 189, 219
- **MCINTYRE, K. W. ; D. J. SHUSTER ; K. M. GILLOOLY ; R. R. WARRIER ; S. E. CONNAUGHTON ; L. B. HALL ; L. H. ARP ; M. K. GATELY ; J. MAGRAM.** Reduced incidence and severity of collagen-induced arthritis in interleukin-12-deficient mice. *Eur. J. Immunol.,* 1996, vol. 26, 2933
- **OKURA, Y. ; K. TAKEDA ; S. HONDA ; H. HANAWA ; H. WATANABE ; M. KODAMA ; T. IZUMI ; Y. AIZAWA ; S. SEKI ; T. ABO.** Recombinant murine interleukin-12 facilitates induction of cardiac myosin-specific type 1 helper T cells in rats. *Circ. Res,* 1998, vol. 82, 1035
- **FONTENOT, J.D. ; M.A. GAVIN ; A. Y. RUDENSKY.** Foxp3 programs the development and function of CD4+CD25+ regulatory T cells. *NatImuncd,* 2003, vol. 4, 330-336
- **RONCAROLO, M.G. ; R. BACCHETTA ; C. BORDIGNON ; S. NARULA ; M. K. LEVINGS.** Type 1 T regulatory cells. *Immunol Rev,* 2001, vol. 182, 68-79
- **STOCK, P. ; 0. AKBARI ; G. BERRY ; G. J. FREEMAN ; R. H. DEKRUYFF ; D. T. UMETSU.** Induction of T helper type 1-like regulatory cells that express 15 Foxp3 and protect against airway hyper-reactivity. *Nat Immunol,* 2004, vol. 5, 1149-1156
- **KHATTRI, R. ; D. KASPROWICZ ; T. COX ; M. MORTRUD ; M. W. APPLEBY ; M. E. BRUNKOW ; S.F. ZIEGLER ; F. RAMSDELL.** The Amount of Scurfin Protein Determines Peripheral T Cell Number and Responsiveness. *JImmunol,* 2001, vol. 167, 6312-6320
- **KHATTRI, R. ; T. COX ; S. A. YASAYKO ; F. RAMSDELL.** An essential role for Scurfin in CD4+CD25+ T regulatory cells. *Nat IMMI,* 2003, vol. 11101 (4), 337-342
- **SAKAGUCHI S ; SAKAGUCHI N ; ASANO M ; ITOH M ; TODA M.** Immunologic self tolerance maintained by activated T cells expressing IL-2 receptor achains. *J Immunol,* 1995, vol. 155, 1151-64
- **SAKAGUCHI S ; TAKAHASHI T ; NISHIZUKA Y.** Study on cellular events in 25 postthymectomy autoimmune oophoritis in mice. I. Requirement of Lyt-1 effector cells for oocytes damage after adoptive transfer. *J Exp Med,* 1982, vol. 156, 1565-76
- **ASANO M ; TODA M ; SAKAGUCHI N ; SAKAGUCHI S.** Autoimmune disease as a consequence of developmental abnormality of a T cell subpopulation. *J Exp Med,* 1996, vol. 184, 387-96
- **NIELSEN J ; HOLM T L ; CLAESSON M H.** CD4+CD25+ regulatory T cells: II. Origin, disease models and clinical aspects. *APHIS,* 2004, vol. 112, 642-50

- **KOHMAP ; CARPENTIER PA ; ANGER HA ; MILLER SD.** Cutting edge: CD4+CD25+ regulatory T cells suppress antigen-specific autoreactive immune responses and central nervous system inflammation during active experimental autoimmune encephalomyelitis. *J Immunol.,* 2002, vol. 169, 4712-4716
- **ZHANG X ; KOLDZIC DN ; IZIKSON L et al.** IL-10 is involved in the suppression of experimental autoimmune encephalomyelitis by CD25(+)CD4(+) regulatory T cells. *Int Immunol.,* 2004, vol. 16, 249-256
- **MOTTET C ; UHLIG HH ; POWRIE F.** Cutting edge: cure of colitis by CD4+CD25+ regulatory T cells. *J Immunol.,* 2003, vol. 170, 3939-3943
- **TANG Q ; HENRIKSEN KJ ; BI M et al.** In vitro-expanded antigen-specific regulatory T cells suppress autoimmune diabetes. *J Exp Med.,* 2004, vol. 199, 1455-1465